# EUROPEAN PATENT APPLICATION

(11) **EP 4 563 177 A1**
(43) Date of publication of application: **04.06.2025**
(21) Application number: 23862099.1
(22) Date of filing: 31.07.2023
(51) Int. Cl.: A61M 16/00, A61M 16/01

(54) **ANESTHESIA MACHINE AND VENTILATION CONTROL METHOD**

(30) Priority: 06.09.2022 WO PCT/CN2022/117392
(71) Applicant: Shenzhen Mindray Bio-Medical Electronics Co., Ltd., Shenzhen, Guangdong 518057 (CN)
(72) Inventor: HUANGFU, Yong, Shenzhen, Guangdong 518057 (CN); ZHOU, Xiaoyong, Shenzhen, Guangdong 518057 (CN); CHEN, Yiwei, Shenzhen, Guangdong 518057 (CN); YANG, Shuwang, Shenzhen, Guangdong 518057 (CN); CAI, Kun, Shenzhen, Guangdong 518057 (CN)
(74) Representative: KIPA AB
(86) International application number: PCT/CN2023/110428
(87) International publication number: WO 2024/051399

(57) **Abstract**

The present invention provides an anesthesia machine and a ventilation control method, wherein a breathing circuit delivers a second gas with an anesthetic to a patient so as to provide anesthesia breathing support for the patient. A ventilation module receives a first gas so as to provide breathing support for the patient at a first ventilation frequency by using the first gas. The anesthesia machine provides, according to a breathing support mode received by a human-computer interaction device, anesthesia breathing support for the patient and/or provides breathing support for the patient. Therefore, the anesthesia machine can be used to provide corresponding ventilation support for a patient as needed in a clinical scenario. The anesthesia machine has a widened range of applications and needs no additional apparatus.

## Description

This disclosure claims partial priority to a PCT application No. PCT/CN2022/117392, filed on September 6, 2022, titled "ANESTHESIA MACHINE", which PCT application No. PCT/CN2022/117392, further claims priority to a Chinese patent application No. 202111040338.6, titled "ANESTHESIA MACHINE" , filed on September 6, 2021, but has added some new technical solutions. Accordingly, this disclosure claims a priority of said new technical solutions which are first submitted by the PCT application No. PCT/CN2022/117392, on its application date. The contents of the PCT application No. PCT/CN2022/117392 are incorporated herein by reference in their entirety.

### TECHNICAL FIELD

The disclosure relates to a technical field of medical device, and more particularly to an anesthesia machine and a ventilation control method.

### BACKGROUND

Anesthesia machines are mainly configured to provide oxygen, anesthesia, and respiratory support to patients during surgery. During the ventilation and anesthesia processes, the anesthesia machine delivers an anesthetic mixture to the patient while receiving an exhaled gas of the patient. In order to save anesthetic gas and improve a comfort degree of the patient when inhaling gas, the exhaled gas is generally recycled. In the circulatory and re-respiratory system, part of the exhaled gas of the patient is re-inhaled as an inhaled gas. In this way, the anesthetic, as a relatively expensive gas component, is retained to the greatest extent, which plays an important role in reducing environmental pollution and saving costs at the same time. Exhaled gas of the patient contains CO₂, which can cause acidosis if it is directly re-inhaled, so the CO₂ needs to be removed before recycling the exhaled gas. A common method is to use a CO₂ absorbent (soda lime) to react with CO₂ to remove CO₂. At the same time, the reaction generates water and heat, which is beneficial to maintaining the temperature and humidity of the gas inhaled by the patient.

For the above reasons, it is necessary to connect an airway system of the anesthesia machine to the respiratory system of the patient and ensure gas tightness, so as to implement effective ventilation and anesthesia. The common method is to insert a tracheal tube into trachea of the patient through endotracheal intubation, so as to connect the anesthesia machine and the trachea of the patient. At the same time, a balloon at the end of the tracheal tube can ensure the gas tightness of this connection and prevent damage to an inner wall of the trachea.

However, in some surgeries for pharyngeal, tracheal, bronchi or lungs, endotracheal intubation cannot be used because it blocks a surgical site, making the surgical operation impossible, or because this ventilation method causes the surgical site to fluctuate periodically according to inhalation and expiration of the ventilation, which affects the surgical effect. Usually when encountering surgical situations like this, additional high-frequency jet device (such as high-frequency jet ventilator) is required. Such device is rarely equipped in anesthesia operation rooms, and the frequency of adding and using the high-frequency jet device is not high, which causes problems in multi-device management. Therefore, many hospitals do not have the conditions for this kind of surgery and can only transfer to other hospitals for surgical treatment, or can only use other compromise methods, which greatly reduces the surgical effect and even brings surgical risks.

Therefore, existing anesthesia machines have limited usage scenarios.

### SUMMARY

This disclosure mainly provides an anesthesia machine and a ventilation control method, so as to improve an application scope of the anesthesia machine.

One embodiment provides an anesthesia machine, including: a housing, a monitoring apparatus for flow amount, a respiratory loop, a first ventilation control apparatus and a human-machine interaction apparatus;
the monitoring apparatus for flow amount is configured to regulate a flow amount of a first gas; wherein after the flow amount of said first gas is regulated by the monitoring apparatus for flow amount, said first gas is delivered to an anesthetic delivery apparatus and then mixed with an anesthetic which is provided by the anesthetic delivery apparatus, so as to obtain a second gas;
the respiratory loop is configured to receive the second gas and deliver the second gas to a patient;
the first ventilation control apparatus is configured to control the respiratory loop to deliver the second gas to said patient, so as to provide anesthesia respiratory support for said patient;
the human-machine interaction apparatus is configured to receive, from a user, a respiratory support mode for a patient which is provided by the anesthesia machine;
wherein the anesthesia machine further includes:
   a ventilation module, which is configured to receive a first gas and provide respiratory support for a patient, by using said first gas at a first ventilation frequency; wherein at least a portion of the ventilation module is arranged inside an accommodation space which is formed by the housing, wherein said portion of the ventilation module and the anesthesia machine form a whole;
   the anesthesia machine is further configured to enable the anesthesia machine to provide the anesthesia respiratory support for a patient and/or provide the respiratory support for a patient, according to the respiratory support mode which is received by the human-machine interaction apparatus.

One embodiment provides an anesthesia machine, including: a monitoring apparatus for flow amount, a respiratory loop, a first ventilation control apparatus and a human-machine interaction apparatus;
the monitoring apparatus for flow amount is configured to regulate a flow amount of a third gas; wherein after the flow amount of the third gas is regulated by the monitoring apparatus for flow amount, the third gas is delivered to an anesthetic delivery apparatus and then mixed with an anesthetic, so as to obtain a second gas;
the respiratory loop is configured to receive the second gas and deliver the second gas to a patient;
the first ventilation control apparatus is configured to control the respiratory loop to deliver the second gas to said patient, so as to provide anesthesia respiratory support for said patient;
the human-machine interaction apparatus is configured to receive, from a user, a respiratory support mode for a patient which is provided by the anesthesia machine;
wherein the anesthesia machine further includes:
   a ventilation module, which is configured to receive a first gas and provide respiratory support for a patient of multiple respiratory cycles, by using said first gas at a first ventilation frequency; wherein the ventilation module is configured to deliver said first gas to a patient at a variable flow rate within a single respiratory cycle;
   the anesthesia machine is further configured to enable the anesthesia machine to provide the anesthesia respiratory support for a patient and/or provide the respiratory support for a patient, according to the respiratory support mode which is received by the human-machine interaction apparatus.

One embodiment provides an anesthesia machine, including:
an anesthesia branch, which is configured to receive a first gas, mix said first gas with an anesthetic to obtain a second gas, and use the second gas to provide anesthesia respiratory support for a patient at a third ventilation frequency;
a ventilation module, which is configured to receive a first gas and provide respiratory support for a patient, by using said first gas at a first ventilation frequency; wherein the ventilation module includes a gas delivery branch and an output port; the gas delivery branch is configured to receive said first gas; wherein the ventilation module further includes a second control valve, which is configured to enable said first gas inside the gas delivery branch to form a gas flow at a first ventilation frequency; or, the ventilation module further includes a first control valve and a third control valve, which together enable said first gas inside the gas delivery branch to form a gas flow at a first ventilation frequency; the output port is configured to output the gas flow at the first ventilation frequency; and
a housing, wherein at least a portion of the ventilation module is arranged inside an accommodation space which is formed by the housing, wherein said portion of the ventilation module and the anesthesia machine form a whole.

One embodiment provides an anesthesia machine, including: a gas source interface, a respiratory loop, and a first ventilation control apparatus; wherein one end of the gas source interface is connected with a gas source, the other end of the gas source interface is connected with an anesthetic delivery apparatus; wherein the anesthetic delivery apparatus is configured to mix a gas, which is provided by the gas source, with an anesthetic, and deliver the mixed gas to the respiratory loop;
the first ventilation control apparatus is configured to control the respiratory loop to deliver the mixed gas to a patient, so as to provide anesthesia respiratory support for said patient;
wherein the anesthesia machine further includes:
   a ventilation module, which is configured to provide respiratory support for a patient, by using the gas which is provided by the gas source; wherein the ventilation module includes a gas delivery branch, which is configured to receive the gas which is provided by the gas source, and provide the respiratory support for said patient, at a ventilation frequency which is higher than 50 times/minute.
   One embodiment provides an anesthesia machine, including: a gas source interface, a respiratory loop, and a first ventilation control apparatus; wherein one end of the gas source interface is connected with a gas source, the other end of the gas source interface is connected with an anesthetic delivery apparatus; wherein the anesthetic delivery apparatus is configured to mix a gas, which is provided by the gas source, with an anesthetic, and deliver the mixed gas to the respiratory loop;
   the first ventilation control apparatus is configured to control the respiratory loop to deliver the mixed gas to a patient, so as to provide anesthesia respiratory support for said patient;
   wherein the anesthesia machine further includes:
      a ventilation module, which is connected with a jet accessory, and configured to receive and regulate a gas which is outputted by the gas source interface, so as to enable said gas to provide respiratory support for a patient in a jet gas flow after passing through the jet accessory.

One embodiment provides an anesthesia machine, including: a gas source interface, a respiratory loop, and a human-machine interaction apparatus;
wherein one end of the gas source interface is connected with a gas source, the other end of the gas source interface is connected with an anesthetic delivery apparatus; wherein the anesthetic delivery apparatus is configured to mix a third gas, which is provided by the gas source with an anesthetic, and deliver to the respiratory loop a second gas which is obtained after the mixing;
the respiratory loop is configured to deliver the second gas to a patient, so as to provide anesthesia respiratory support for said patient;
the human-machine interaction apparatus is configured to receive, from a user, a respiratory support mode for a patient which is provided by the anesthesia machine;
wherein the anesthesia machine further includes:
   a communication module, which is configured to communicate with an external ventilation apparatus; wherein the ventilation apparatus is configured to provide respiratory support for a patient, by using a first gas at a first ventilation frequency; wherein the first ventilation frequency is not zero;
   the anesthesia machine is further configured to enable the anesthesia machine to provide the anesthesia respiratory support for a patient, and/or configured to control, through the communication module, the ventilation apparatus to provide the respiratory support for a patient, according to the respiratory support mode which is received by the human-machine interaction apparatus.

An embodiment provides a ventilation control method for an anesthesia machine, including:
receiving, from a user, a respiratory support mode which is provided by the anesthesia machine;
when the received respiratory support mode is to provide anesthesia respiratory support: receiving a third gas, controlling the third gas to mix with an anesthetic to obtain a second gas, controlling the second gas to enter into a respiratory loop of the anesthesia machine, and controlling the respiratory loop to output the second gas, so as to provide anesthesia respiratory support for a patient;
when the received respiratory support mode is to provide respiratory support through a ventilation module: receiving a first gas, and controlling the ventilation module to provide the respiratory support for a patient, by using the first gas at a flow rate in a single respiratory cycle, which flow rate is not fixed.

According to the anesthesia machine of the above embodiment, a respiratory loop delivers a second gas containing anesthetic to a patient, thereby providing anesthesia respiratory support for said patient; a ventilation module receives a first gas to provide respiratory support for a patient by using the first gas at a first ventilation frequency, and the anesthesia machine provides the anesthesia respiratory support and/or the respiratory support for a patient according to a respiratory support mode received by the human-machine interaction apparatus. Therefore, the anesthesia machine can provide corresponding ventilation support to a patient according to requirements of clinical scenarios, which expands an application scope of the anesthesia machine without requiring an additional device.

### BRIEF DESCRIPTION OF THE DRAWINGS

FIG. 1 is a structural block diagram of an existing anesthesia machine.
FIG. 2 is a structural block diagram of an embodiment of an anesthesia machine provided by this disclosure.
FIG. 3 is a structural block diagram of an embodiment of an anesthesia machine provided by this disclosure.
FIG. 4 is a structural block diagram of an embodiment of an anesthesia machine provided by this disclosure.
FIG. 5 is a structural block diagram of an embodiment of a ventilation module in an anesthesia machine provided by this disclosure.
FIG. 6 is a gas path diagram of an embodiment of a ventilation module in an anesthesia machine provided by this disclosure.
FIG. 7 is a gas path diagram of an embodiment of a ventilation module in an anesthesia machine provided by this disclosure.
FIG. 8 is a gas path diagram of an embodiment of a ventilation module in an anesthesia machine provided by this disclosure.
FIG. 9 is a pressure waveform diagram displayed on a display in an anesthesia machine provided by this disclosure.
FIG. 10 is a diagram of a main monitoring interface on a display in an anesthesia machine provided by this disclosure.
FIG. 11 is a structural block diagram of a monitoring apparatus for flow amount in an embodiment shown in FIG. 3.
FIG. 12 is a structural block diagram of a monitoring apparatus for flow amount in an embodiment shown in FIG. 4.
FIG. 13 is a gas path diagram of an embodiment of a jet apparatus in an anesthesia machine provided by this disclosure.
FIG. 14 is a structural block diagram of an embodiment of an anesthesia machine provided by this disclosure.
FIG. 15 is a flow chart of an embodiment of a ventilation control method provided by this disclosure.
FIG. 16 is a structural block diagram of an embodiment of an anesthesia machine provided by this disclosure.

### DESCRIPTION OF THE PREFERRED EMBODIMENTS

This disclosure is further described in detail below through specific embodiments in combination with the accompanying drawings. In different embodiments, similar elements adopt associated similar reference numbers. In the following embodiments, many details are described in order to make this disclosure better understood. However, one skilled in the art can easily recognize that some of the features can be omitted in different cases, or can be replaced by other elements, materials and methods. In some cases, some operations related to this disclosure are not shown or described in the description, in order to avoid the core part of this disclosure being inundated by too many descriptions. For one skilled in the art, it is not necessary to describe these related operations in detail, as they can fully understand the relevant operations according to the description in this disclosure and the general technical knowledge in the art.

In addition, the features, operations, or characteristics described in the specification may be combined in any appropriate manner to form various embodiments. At the same time, the steps or actions in the method description can also be exchanged or regulated in order in a manner obvious to one skilled in the art. Therefore, the various sequences in the description and the drawings are only for the purpose of clearly describing a certain embodiment, and do not mean that they are necessary sequences. Unless it is otherwise specified that one of the sequences must be followed.

The terms "first", "second", etc. in the specification are operable to distinguish different objects, rather than to describe a specific order. In addition, the terms "connection", and "linkage" mentioned in this disclosure include direct and indirect connection (linkage), unless otherwise specified.

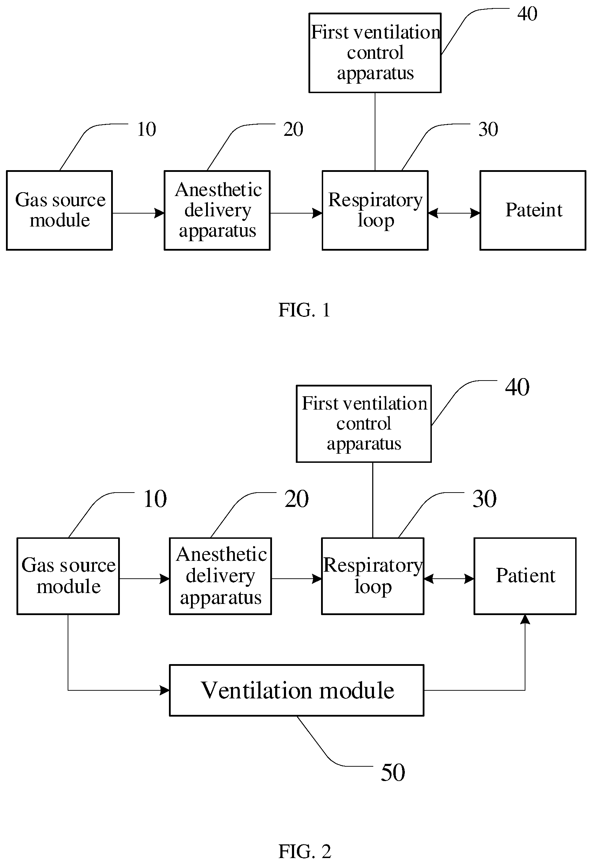

As shown in FIG. 1, an existing anesthesia machine generally includes a gas source module 10, an anesthetic delivery apparatus 20, a respiratory loop 30 and a first ventilation control apparatus 40. The gas source module 10 is connected with the respiratory loop 30 through the anesthetic delivery apparatus 20, that is, the gas source module 10, the respiratory loop 30 and the anesthetic delivery apparatus 2 are connected in sequence in a gas path.

The gas source module 10 is configured to provide a gas which is required by the anesthesia machine, that is, to provide a first gas, such as one or more of oxygen, air and laughing gas (nitrous oxide), because it is necessary to provide respiratory support for a patient. Therefore, the gas provided contains at least oxygen. That is, the first gas may be oxygen, air, a mixture of oxygen and air, a mixture of air and nitrous oxide, a mixture of oxygen and nitrous oxide, or a mixture of oxygen, air and nitrous oxide, etc. The gas source module 10 may be a gas source interface which is connected with an external gas source. The external gas source provides the gas required by the anesthesia machine to the anesthesia machine through the gas source interface. The gas source module 10 can also be an air compressor, an oxygen generator, etc., and generate the first gas, which is required by the anesthesia machine by itself. Subsequent embodiments are described by using the gas source interface as an example.

The anesthetic delivery apparatus 20 is configured to mix the first gas, which is provided by the gas source module 10, with an anesthetic, control an anesthetic concentration of the mixed gas (second gas), and deliver the mixed gas (second gas) to the respiratory loop 30. The anesthetic delivery apparatus 20 includes a vaporizer. The anesthetic delivery apparatus 20 may be a part of the anesthesia machine, that is, the anesthesia machine is equipped with the anesthetic delivery apparatus 20 after leaving a factory, the anesthetic delivery apparatus may also be an accessory of the anesthesia machine, which is installed at the anesthesia machine when using the anesthesia machine.

The respiratory loop 30 is a gas path, which connects the anesthetic delivery apparatus 20 and a patient, receives the second gas, and delivers the second gas to the patient, which can also recycle an exhaled gas of the patient to save anesthetic and reduce environmental pollution. The respiratory loop 30 can include various connection tubes and accessories. The accessories can be an endotracheal tube, an endotracheal tube with a balloon at its end, etc. A gas purification apparatus may be provided in the respiratory loop 30, and configured to remove at least part of carbon dioxide exhaled by the patient into the respiratory loop. For example, CO₂ absorbent (soda lime) can be installed in the gas purification apparatus. The CO₂ absorbent reacts with CO₂ to achieve the purpose of removing CO₂. At the same time, the reaction generates water and heat, which is beneficial to maintaining the temperature and humidity of inhaled gas the patient.

The first ventilation control apparatus (i.e., an anesthesia ventilation control apparatus) 40 is configured to control the respiratory loop 30 to deliver the mixed gas (second gas) to a patient, thereby providing anesthesia respiratory support for said patient. Providing anesthesia respiratory support gives a respiratory cycle to a patient, so the anesthesia respiratory support provided to the patient has a certain ventilation frequency (for ease of distinction, it is called as a third ventilation frequency). That is, the first ventilation control apparatus 40 controls the respiratory loop 30 to periodically deliver the second gas to a patient, thereby providing periodic anesthesia respiratory support for the patient; "periodic" here refers to a respiratory cycle which is formed by ventilation at the third ventilation frequency. In other words, the first ventilation control apparatus 40 controls the respiratory loop 30 to provide anesthesia respiratory support for the patient by using the second gas at the third ventilation frequency. Surgery takes time, so anesthesia respiratory support usually lasts for a period of time, and has multiple respiratory cycles.

The first ventilation control apparatus 40 can control anesthesia ventilation automatically or manually (such as a balloon). For example, the first ventilation control apparatus 40 may include a plurality of valves and a board card for driving the plurality of valves. The board card controls the plurality of valves to periodically deliver the second gas to the patient, thereby providing periodic anesthesia respiratory support for a patient.

During the operation of the anesthesia machine, the first gas is provided by the gas source module 10, and a flow amount of the first gas is regulated through the monitoring apparatus for flow amount, then an addition and concentration adjustment of the anesthetic are implemented by an evaporator, so as to form a second gas which enters into the respiratory loop 30. The first ventilation control apparatus 40 performs a ventilation control and delivers the second gas to the patient. The exhaust gas exhaled by the patient is purified by the gas purification apparatus and then discharged or recycled. During the above process, the anesthesia machine also monitors a machine state and patient parameter(s) to ensure patient safety and send out abnormal alarm.

This disclosure creatively integrates a ventilation module which provides respiratory support on an anesthesia machine, so as to enable the anesthesia machine to provide respiratory support through the ventilation module and/or to provide anesthesia respiratory support through the first ventilation control apparatus. For example, in some specific scenarios, such as when the inhalation anesthesia function of the anesthesia machine does not need to be used, the ventilation module of the anesthesia machine can be configured to provide respiratory support for the patient, which improves the application scope of the anesthesia machine, eliminates the need to use the additional device, saves management issue of multiple devices. Wherein, the respiratory support provided by the ventilation module can be high-frequency ventilation (for example, the ventilation module includes a gas delivery branch, which receives a gas provided by an external gas source and provides respiratory support for a patient at a ventilation frequency, which is not lower than 50 times/minute (50bpm) or not lower than 3 Hz) or low-frequency ventilation, etc. The ventilation mode of the ventilation module can be jet ventilation or non-jet ventilation. When providing the jet ventilation, the ventilation module can further provide high-frequency jet ventilation. Detailed description is given below through some examples.

As shown in FIG. 2, the anesthesia machine provided by this disclosure adds a ventilation module 50, which is connected with the gas source module 10 on the basis of the existing anesthesia machine. The gas source module 10 and the ventilation module 50 can be in direct connection or indirect connection, in short, just in connection. The ventilation module 50 is configured to provide respiratory support for a patient, by using the first gas (such as oxygen and/or air) provided by the gas source module 10. That is, the ventilation module 50 is configured to receive the first gas, so as to provide respiratory support for the patient, by using using the first gas. Likewise, the respiratory support provided by the ventilation module 50 enables the patient to have a respiratory cycle, so that the respiratory support provided by the ventilation module 50 to the patient has a certain ventilation frequency (for ease of distinction, referred to as a first ventilation frequency). That is, the ventilation module 50 receives the first gas, so as to provide periodic respiratory support for a patient; "periodic" here refers to a respiratory cycle which is formed by ventilation at the first ventilation frequency. In other words, the ventilation module 50 receives the first gas, so as to provide respiratory support for a patient, by using the first gas at a first ventilation frequency. Usually, respiratory support is provided to a patient for a period of time, such that the respiratory support has multiple respiratory cycles, that is, the ventilation module 50 uses the first gas at the first ventilation frequency to provide a patient respiratory support of multiple respiratory cycles.

It can be seen that, when no inhalation anesthesia is required, the anesthesia machine can also be configured to ventilate the patient, which improves the application scope of the anesthesia machine without requiring additional device(s). In some usage scenarios, the human-machine interaction apparatus provided by the anesthesia machine can be configured to receive a respiratory support mode, which is provided for the patient by the anesthesia machine. For example, trigger button(s) for different respiratory support modes can be provided on a display interface of the human-machine interaction apparatus. The anesthesia machine can decide, based on the respiratory support mode received by the human-machine interaction apparatus, whether to use the ventilation module to provide periodic respiratory support for a patient, or to use the respiratory loop to provide periodic anesthesia respiratory support for a patient; or to simultaneously use both of the ventilation module and the respiratory loop to provide respiratory support for different patients. "Periodic respiratory support" and "periodic anesthesia respiratory support" mentioned in this disclosure represent two different respiratory support modes. What they have in common is that a gas delivery is provided to a patient at a variable flow rate within one respiratory cycle; what they have in difference is that a gas delivered under periodic anesthesia respiratory support includes anesthetics, while a gas delivered under periodic respiratory support does not contain anesthetics. Specifically, providing respiratory support for a patient by the ventilation module 50 means that a first gas is delivered to a patient at a variable flow rate during a single respiratory cycle, that is, the first gas is delivered to the patient at two flow rates, one is large and the other one is small, during a single respiratory cycle, and the small flow rate may be zero or not. When the small flow rate is zero, no gas is delivered to the patient for at least a portion of time during a single respiratory cycle. Providing anesthesia respiratory support for a patient by the first ventilation control apparatus means that a second gas is delivered to the patient at a variable flow rate during single respiratory cycle, that is, the second gas is delivered to a patient at two flow rates, one is large and the other one is small, during a single respiratory cycle, and the small flow rate can be zero or not. **In** this embodiment, since the flow rates provided by the two respiratory support modes vary in a single respiratory cycle, the first ventilation frequency and the third ventilation frequency are both non-zero ventilation frequencies, or in other words, values of the first ventilation frequency and the third ventilation frequency are greater than zero.

The ventilation module 50 can be connected with various accessories, such as a jet accessory, a nasal plug, a nasal mask, a mask, etc. The doctor can connect an appropriate accessory and perform corresponding ventilation method according to actual condition of the patient and ventilation requirements, which is very convenient.

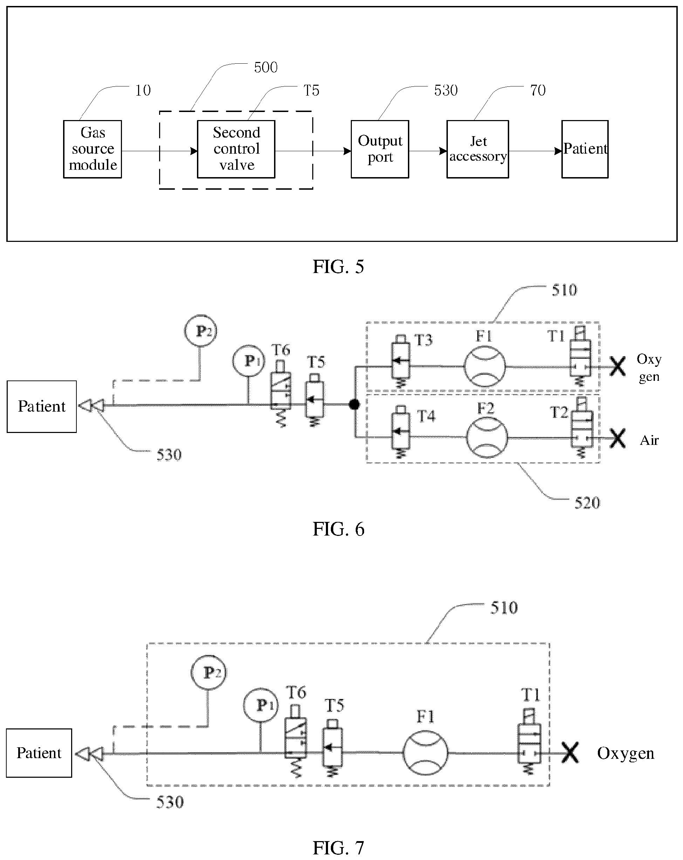

The ventilation module 50 can be implemented in a variety of ways, as shown in FIGS. 6, 7 and 8. In provided periodic respiratory support, the specific ventilation method can be high-frequency ventilation and/or jet ventilation, wherein the jet ventilation can be high-frequency jet ventilation, or low-frequency jet ventilation. The expression "high frequency/jet ventilation" as used herein refers to either high frequency ventilation or jet ventilation. Below are some examples to illustrate.

### First Embodiment

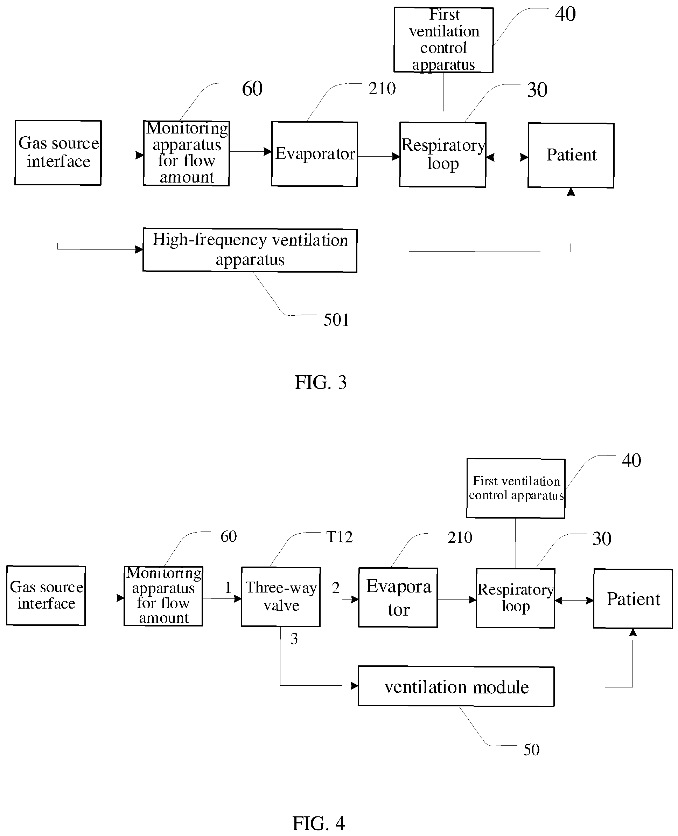

In this embodiment, a ventilation mode provided by the ventilation module 50 is high-frequency ventilation. For example, as shown in FIGS. 3 and 4, the ventilation module 50 includes a high-frequency ventilation apparatus 501.

The high-frequency ventilation device 501 is configured to ventilate a patient at a ventilation frequency of at least 50 times/minute, thereby providing periodic respiratory support for the patient. Of course, the patient can also be ventilated at a higher ventilation frequency at or over 3 Hz. As shown in FIG. 5, the high-frequency ventilation apparatus 501 may include a gas delivery branch 500, a second control valve T5 and an output port 530. The high-frequency ventilation apparatus 501 is configured to receive the first gas through the gas delivery branch 500. The second control valve T5 is arranged inside the gas delivery branch 500 and is controlled to switch an amount of flow at a switching frequency of at least 50 times/minute, so as to enable a first gas which is outputted from the output port 530 to form a high-frequency gas flow. Of course, the amount of flow can also be switched at a switching frequency which is at or over 3 Hz. The expression "high-frequency gas flow" used in this disclosure refers to a gas flow whose output frequency is higher than a certain critical value, for example, a gas flow whose output frequency is higher than or equal to 3 Hz, or greater than or equal to 50 times/minute.

The ventilation frequency (switching frequency for an amount of flow or switching frequency of opening degree for a valve) of the second control valve T5 can be preset. The second control valve T5 is used for high-frequency ventilation control for example, switching between two different valve flow amounts, one is large and the other one is small, at a high frequency (switching of the opening degree for a valve results in a change of a flow rate in a single respiratory cycle), which frequency can be above 50 times/minute, for example, between 3-20Hz, so as to enable the first gas to form a gas flow of high-frequency pulse, which gas is capable of providing for a patient high-frequency jet ventilation after passing through a subsequent jet accessory, or providing high-frequency ventilation for a patient without subsequently connecting a jet accessory. The second control valve T5 may be an electromagnetic valve, an electromagnetic servo valve, an electromagnetic switch valve or an electromagnetic proportional valve, etc.

In this embodiment, the high-frequency ventilation apparatus 501 provides periodic respiratory support at a first ventilation frequency, and the first ventilation control apparatus 40 controls the respiratory loop 30 to provide periodic anesthesia respiratory support at a third ventilation frequency. The first ventilation frequency is higher than the third ventilation frequency. For example, the first ventilation frequency is greater than or equal to 50 times/minute, or higher than or equal to 3 Hz, and the third ventilation frequency is less than 50 times/minute, less than 3 Hz.

The high-frequency ventilation apparatus 501 can be implemented in various ways, as shown in FIG. 6, FIG. 7, and FIG. 8, which are introduced one by one below.

As shown in FIG. 7, the first gas may include oxygen. The gas source module 10 includes an oxygen unit which provides oxygen. For example, the oxygen unit is an oxygen interface or an oxygen generator. The oxygen interface is connected with an external oxygen source. For example, the oxygen interface is connected with an oxygen delivery pipeline, an oxygen bottle, in the hospital, etc. The gas delivery branch 500 includes an oxygen branch 510, that is, the ventilation module 50 or the high-frequency ventilation apparatus 501 includes: the oxygen branch 510, the second control valve T5 and the output port 530. The oxygen branch 510 is connected with the oxygen unit, for example, the oxygen branch 510 and the oxygen unit are directly connected. The oxygen unit is connected with the output port 530 through the oxygen branch 510. The second control valve T5 is arranged inside the oxygen branch 510. The second control valve T5 switches a flow amount at a preset frequency (for example, at least 3 Hz), which is capable of controlling a flow amount of oxygen inside the oxygen branch 510, enabling the oxygen outputted by the output port 530 to form a high-frequency gas flow, thereby providing periodic high-frequency ventilation for a patient. The oxygen branch 510 may also be provided with a first switch valve T1, so as to control an on/off state of the oxygen branch 510.

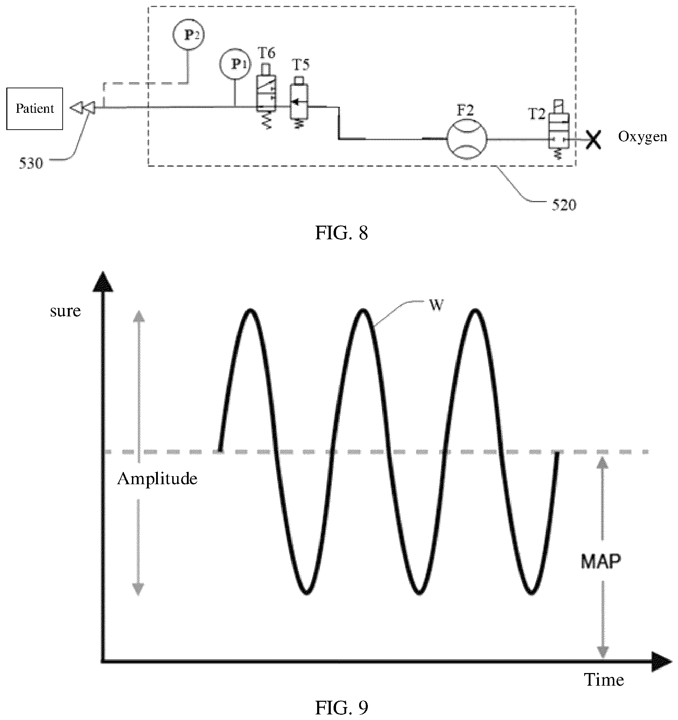

As shown in FIG. 8, the first gas may also include air. The gas source module 10 includes an air unit, which provides air. For example, the air unit is an air interface, which is connected with an external gas source, such as an air delivery duct in hospital. For another example, the air unit is a turbine, which obtains air from external atmospheric environment. The gas delivery branch 500 includes an air branch 520 , that is, the ventilation module 50 or the high-frequency ventilation apparatus 501 includes: the air branch 520, the second control valve T5 and the output port 530. The air branch 520 is connected with the air unit, for example, the air branch 520 and the air unit are directly connected. The air unit is connected with the output port 530 through the air branch 520. The second control valve T5 is arranged inside the air branch 520. The second control valve T5 switches a flow amount at a preset frequency (for example, at least 3 Hz), which is capable of controlling a flow amount of air inside the air branch 520, enabling the air outputted by the output port 530 to form a high-frequency gas flow, thereby providing periodic high-frequency ventilation for a patient. The air branch 520 may also be provided with a second switch valve T2, so as to control an on/off state of the air branch 520.

In the embodiment shown in FIGS. 7 and 8, the second control valve T2 switches the flow amounts at a frequency that can realize high-frequency ventilation. In the process of high-frequency regulation for opening degrees of the valve, flow amounts inside the oxygen branch and the air branch are controlled by the opening degree, and the ventilation frequencies of gases in the oxygen branch and the air branch can be controlled through the switching frequency.

As shown in FIG. 6, the first gas may also include oxygen and air. The gas source module 10 includes an oxygen unit which provides oxygen, and an air unit. The gas delivery branch 500 includes an oxygen branch 510 and an air branch 520, that is, the ventilation module 50 or the high-frequency ventilation apparatus 501 includes: the oxygen branch 510, the air branch 520, the second control valve T5 and the output port 530. The oxygen branch 510 is connected with the oxygen unit, for example, the oxygen branch 510 and the oxygen unit are directly connected. The air branch 520 is connected with the air unit, for example, the air branch 520 and the air unit are directly connected. The oxygen branch 510 and the air branch 520 are converged with each other, and then connected with the output port 530 through the second control valve T5. The oxygen branch 510 is provided with a first control valve T3, which is configured to control the flow amount of the oxygen inside the oxygen branch 510. The oxygen branch 510 can also be provided with a first switch valve T1 which is configured to control an on/off state of the oxygen branch 510, wherein he first switch valve T1 plays a safety protection role. The air branch 520 is provided with a third control valve T4, which is configured to control the flow amount of the air inside the air branch 520. The air branch 520 may also be provided with a second switch valve T2 which is configured to control an on/off state of the air branch 520, wherein the second switch valve T2 plays a safety protection role. The first control valve T3 may be a proportional valve or a flow valve, and the third control valve T4 may be a proportional valve or a flow valve. The first control valve T3 and the third control valve T4 can jointly control a total flow amount and an oxygen concentration of the oxygen-air mixed gas. The second control valve T5 switches a flow amount at a preset frequency (for example, at least 3Hz), so as to form a high-frequency gas flow from the converged oxygen-air mixed gas. The high-frequency gas flow is outputted through the output port 530, so as to provide high-frequency ventilation for the patient. In some embodiments, as an alternative to the embodiment shown in FIG. 6, the second control valve T5 may not be provided, but the first control valve T3 and the third control valve T4 may jointly realize the high-frequency ventilation control. That is, the first control valve T3 is used both to control the flow amount of the oxygen inside the oxygen branch 510, and to enable the oxygen inside the oxygen branch to form a high-frequency gas flow, thereby realizing the high-frequency ventilation. The third control valve T4 is used both to control the flow amount of the air inside the air branch 520, and to enable the air inside the air branch 520 to form a high-frequency gas flow, thereby realizing the high-frequency ventilation. In this alternative embodiment, the first control valve T3 and the third control valve T4 can switch an amount of flow at a high-frequency ventilation frequency; can control flow amounts of the gases inside the air branch and the branch oxygen according to the opening degrees of the valves, during the process of high-frequency regulation for opening degrees of the valves; and can control ventilation frequencies of gases in the oxygen branch and the air branch through the switching frequency. Therefore, as an alternative to the embodiment shown in FIG. 6, by controlling two control valves (that is, the first control valve T3 and the third control valve T4) to switch an amount of flow at a first ventilation frequency (such as greater than or equal to 50 times/minute), between two different valve flow amounts, one is large and the other one is small, such as switching between opening the valve and partially closing the valve at a hight frequency or switching between opening the valve and completely closing the valve at a hight frequency, so as to enable an oxygen-air mixed gas to form a high-frequency gas flow, thereby providing high-frequency ventilation for a patient.

In the embodiment of FIGS. 6 to 8, both the first switch valve T1 and the second switch valve T2 play a safety protection role. When the ventilation module 50 is not in use, if the ventilation module 50 has a gas output, a gas supply of the anesthesia delivery system of the original anesthesia machine and ventilation of the anesthesia ventilator are affected, and meanwhile, losses of oxygen or gas source are also caused. Therefore, the first switch valve T1 and the second switch valve T2 are provided to prevent gas output, when the oxygen control valve T3, the air control valve T4 or the second control valve T5 cannot be closed during a time in which the ventilation module is not in use.

It can be known from the above-mentioned high-frequency ventilation apparatus 501 that the high-frequency gas flow formed at its output port 530 ventilates the patient at a ventilation frequency of at least 50 times/minute. Or in some embodiments, the high-frequency gas flow formed at its output port 530 ventilates the patient in a ventilation frequency at one value between 50-1500 bpm. High-frequency ventilation includes high-frequency positive pressure ventilation, high-frequency oscillation ventilation and high-frequency jet ventilation. When the above-mentioned high-frequency ventilation apparatus is used for high-frequency positive pressure ventilation or high-frequency oscillation ventilation, the ventilation frequency for the patient is at least 3Hz. When the high-frequency ventilation apparatus is used for high-frequency jet ventilation, the ventilation frequency for the patient is at least 50-1500bpm. The high-frequency ventilation provided by the ventilation module 50 can be pure high-frequency ventilation, or high-frequency jet ventilation after adding the jet accessory 70. Specifically, if the output port 530 is not connected with the jet accessory, but to other ventilation accessories (Such as trachea, intubation, mask, nasal mask, etc.), the high-frequency ventilation apparatus 501 simply provides high-frequency ventilation for the patient. If the output port 530 is connected with the jet accessory 70, the high-frequency ventilation apparatus 501 can further provide high-frequency jet ventilation for the patient. Taking the latter as an example, the output port 530 can be connected with a jet accessory, and the high-frequency gas flow, which is outputted by the output port 530, is delivered through the jet accessory to form a gas flow of high-frequency jet. The jet accessory can be provided by a third-party manufacturer, and the user can connect it to the output port 530. Of course, the jet accessory can also a part of anesthesia machine accessories, that is, the anesthesia machine includes a jet accessory which is connected with the high-frequency ventilation apparatus 501. The jet accessory receives the first gas outputted by the high-frequency ventilation apparatus 501 and outputs the first gas in the form of a gas flow of high-frequency jet. That is, the output port 530 outputs a high-frequency gas flow (gas flow of high-frequency pulse), which becomes a gas flow of high-frequency jet after passing through the jet accessory. A ventilation frequency of the gas flow of high-frequency jet for ventilating a patient can be at or over 50 times/minute, or between 50-1500bpm, or at or over 3 Hz, thus providing high-frequency jet ventilation for a patient. For high-frequency oscillation ventilation and high-frequency positive pressure ventilation, the ventilation frequency can be 3Hz or above. For high-frequency jet ventilation, the ventilation frequency can be one value between 50-1500bpm. The jet accessory includes a jet channel, an output aperture of the jet channel is less than 4mm. The jet accessory may include a jet nozzle, a jet needle, or a stent (such as a tracheoscope, a bronchoscope, a scope sheath, etc.). The stent is configured to support human tissue or connect the endotracheal tube to facilitate the surgery of the doctor. A wall of the stent is provided with a hole for the gas jet. (For example, its aperture is less than 4mm).

### Second Embodiment

In this embodiment, the ventilation mode provided by the ventilation module 50 is jet ventilation. The ventilation module 50 is further connected with a jet accessory, and is configured to receive the gas, which is outputted from the gas source interface and regulate the gas, so as to enable the gas to provide respiratory support for a patient in a jet gas flow after passing through the jet accessory. The gas path diagram is still shown in FIGS. 6-8. That is, the ventilation module 50 includes a gas delivery branch 500, a second control valve T5 and an output port 530. The second control valve T5 is arranged inside the gas delivery branch 500 and is configured to control the ventilation module 50, so as to provide periodic respiratory support. The output port 530 is connected with the jet accessory. The ventilation module 50 receives the first gas through the gas delivery branch 500, and the first gas is outputted through the jet accessory 70, which is connected with the output port 530, so as to form a jet gas flow. In some embodiments, the anesthesia machine may include a jet accessory 70 which is connected with the ventilation module 50. The jet accessory 70 receives the first gas, which is outputted by the ventilation module 50 and forms a jet gas flow from the first gas. In other embodiments, the anesthesia machine may not include a jet accessory, and the user can install the jet accessory to the output port 530 of the ventilation module by himself during subsequent use. The expression "jet ventilation" used in this disclosure refers to a following ventilation mode. During a gas delivery phase of one respiratory cycle, a high-speed gas is ejected into a respiratory tract by passing a high-pressure gas through a fine-aperture jet accessor, wherein the high-speed gas uses an entrainment/Venturi effect to supplement a tidal volume. In this disclosure, the term "jet gas flow", which corresponds to jet ventilation, refers to a high-speed gas flow, which is outputted by the jet accessory.

In this embodiment, the ventilation module 50 can have various gas path diagrams, as shown in FIGS. 6, 7 and 8, which are introduced one by one below.

As shown in FIG. 7, the gas source module 10 includes an oxygen unit, and the first gas includes oxygen, which is provided by the oxygen unit. The gas delivery branch 500 includes an oxygen branch 510, that is, the ventilation module includes: an oxygen branch 510, a second control valve T5 and an output port 530. The oxygen unit is connected with the output port 530 through the oxygen branch, and the output port 530 is connected with the jet accessory. The second control valve T5 is arranged inside the oxygen branch 510, and is configured to control a flow amount of oxygen inside the oxygen branch 510 and enable the oxygen, which is outputted from the output port 530, to form a periodic jet gas flow after passing through the jet accessory, thereby providing periodic jet ventilation to the patient. Similarly, the oxygen branch 510 may also be provided with a first switch valve T1, so as to control an on/off state of the oxygen branch 510.

As shown in FIG. 8, the gas source module 10 includes an air unit, and the first gas includes air, which is provided by the air unit. The gas delivery branch 500 includes an air branch 520, that is, the ventilation module 50 includes: an air branch 520, a second control valve T5 and an output port 53. The air unit is connected with the output port 530 through the air branch 520, and the output port is connected with the jet accessory. The second control valve T5 is arranged inside the air branch 520 and is configured to control a flow amount of air inside the air branch 520 and enable the air, which is outputted from the output port 530, to form a periodic jet gas flow after passing through the jet accessory, thereby providing periodic jet ventilation to the patient. Similarly, the air branch 520 may also be provided with a second switch valve T2 so as to control an on/off state of the air branch 520.

In the embodiments of FIGS. 7 and 8, the second control valve T5 switches a flow amount at a certain frequency, and controls flow amounts of the gases inside the air branch and the branch oxygen according to the opening degrees of the valves, during the process of high-frequency regulation for opening degrees of the valves, and controls ventilation frequencies of gases in the oxygen branch and the air branch through a switching frequency, so as to enable the oxygen branch and the air branch to respectively outputs periodic gas flows. When the periodic gas flows from the output port are outputted through the jet channel of the jet accessory, periodic injection ventilation is provided for the patient.

As shown in FIG. 6 , the gas source module 10 includes an oxygen unit which provides oxygen and an air unit which provides air. The first gas includes oxygen provided by the oxygen unit and air provided by the air unit. The gas delivery branch 500 includes an oxygen branch and an air branch, that is, the ventilation module 50 includes: an oxygen branch 510, an air branch 520, a second control valve T5 and an output port 530. The oxygen branch 510 is connected with the oxygen unit, and the air branch 520 is connected with the air unit. The oxygen branch 510 and the air branch 520 are converged with each other, and then connected with the output port 530 through the second control valve T5. The output port 530 is connected with a jet accessory. Wherein the oxygen branch 510 is provided with a first control valve T3, and the air branch 520 is provided with a third control valve T4. The first control valve T3 is configured to control the flow amount of the oxygen inside the oxygen branch 510 , and the third control valve T4 is configured to control the flow amount of air inside the air branch 520. The second control valve T5 is configured to form a periodic jet gas flow, when the converged oxygen-air mixed gas is outputted from the jet accessory, which is connected with the output port 530, thereby providing periodic jet ventilation for the patient.

In some embodiments, as an alternative to the embodiment shown in FIG. 6 , the second control valve T5 may not be provided, but the first control valve T3 and the third control valve T4 may jointly realize the jet control. That is, the first control valve T3 is used both to control the flow amount of the oxygen inside the oxygen branch 510, and to enable the oxygen inside the oxygen branch to form an outputted oxygen flow which is periodic, so as to realize the jet ventilation. The third control valve T4 is used both to control the flow amount of the air inside the air branch 520, and to enable the air inside the air branch to form an outputted air flow which is periodic, so as to realize the jet ventilation. That is, when a mixed gas, which is formed by the periodic oxygen flow of the oxygen branch 510 and the periodic air flow of the air branch 520, is outputted from the jet accessory, which is connected with the output port 530, a periodic jet gas flow is formed.

It can be seen from the embodiments in FIGS. 6-8 that, in this embodiment, the ventilation module 50 provides periodic respiratory support for a patient by performing jet ventilation on the patient. Jet ventilation is one of positive pressure ventilation. Jet ventilation is a ventilation manner that ejects high-pressure gas into the respiratory tract at high speed under an open airway. Jet ventilation has a wide range of ventilation frequencies. **In** order to satisfy the requirements of user with different physiological conditions, jet ventilation can have a variety of ventilation frequencies, such as high-frequency jet ventilation and low-frequency jet ventilation. High frequency and low frequency are relative concepts, which are equivalent to multiple ventilation frequency gears. The frequency of high-frequency jet ventilation is higher than the frequency of low-frequency jet ventilation. In some embodiments, for example, the frequency of high-frequency jet ventilation can be between 50 and 1500 bpm. In other embodiments, the frequency of high-frequency jet ventilation can be above 3 Hz, such as between 3 and 20 Hz. The frequency of low-frequency ventilation can be, for example, between 10-50 bpm. In this disclosure, another difference between jet ventilation and periodic anesthesia respiratory support which is controlled by the first ventilation control apparatus, is that the jet ventilation is positive pressure ventilation under an open airway, while periodic anesthesia respiratory support is applied to a ventilation scenarios of closed airway closure or intubation.

In the existing technology, some anesthesia machines, in addition to providing anesthesia respiratory support to patients, can also provide an oxygen therapy of high-flow amount, that is, deliver oxygen of high-flow amount to the patient, thereby achieving pre-oxygenation of the patient and extending asphyxiation time window of the patient, leaving time for doctors to perform intubation and other operations. Pre-oxygenation does not involve the control of the respiratory cycle, but the newly added ventilation module 50 in this embodiment needs to provide periodic respiratory support for a patient, which involves the control of the respiratory cycle, that is, involves the control of the ventilation frequency.

In one embodiment, the ventilation module 50 may include a second ventilation control apparatus. For example, the second ventilation control apparatus may also include one or more valves and a board card for driving the one or more valves. The one or more valves are provided inside the gas delivery branch (such as the above-mentioned oxygen branch, air branch, etc). The board card controls one or more valves (such as various control valves as shown in FIGS. 6-8) to periodically deliver the first gas to the patient, thereby providing periodic respiratory support for a patient. The board card controls an opening degree of one or more valves at a certain frequency, so as to form a periodic gas flow, so that the ventilation module 50 can provide periodic respiratory support for a patient. Combined with the above embodiments, when the second ventilation control apparatus controls the switching frequencies of the opening degrees of the one or more valves to be above 50 times/minute, the ventilation module 50 can provide high-frequency ventilation for a patient. When the second ventilation control apparatus controls the switching frequencies of the opening degrees of the one or more valves to be 10-1500 bpm, and the ventilation module 50 is connected with a jet accessory, the ventilation module 50 can provide jet ventilation for a patient.

In the above embodiment, the oxygen branch 510 may also be provided with a first flow sensor F1 for monitoring the flow amount of the oxygen branch 510. The air branch 520 may also be provided with a first flow sensor F2 for monitoring the flow amount of the air branch 520. Through these two flow sensors, the flow amounts of oxygen and air can be well monitored.

A safety valve T6 for pressure relief, and at least one pressure sensor for monitoring pressure, are provided between a converging node (black dot in FIG. 6) of the oxygen branch 510 and the air branch 520, and the output port 530. The safety valve T6 can be specifically configured to connect the output port 530 with an exhaust end of the safety valve T6, so as to discharge the gas between the output port 530 and the safety valve T6 through the exhaust end for preventing the patient from barotrauma, when a pressure monitored by the pressure sensor exceeds a preset threshold. The safety valve T6 can be specifically configured to separate airways between the output port 530 and the converging node, when a pressure monitored by the pressure sensor exceeds a preset threshold. For example, the safety valve T6 is a three-way valve, wherein a first port of the three-way valve is connected with the output port 530, a second port of the three-way valve is connected with the converging node, and a third port of the three-way valve is the exhaust port. Under normal circumstances, the first port of the three-way valve is connected (communicated) with the second port of the three-way valve, while the first port of the three-way valve is disconnected from the exhaust port, and the second port of the three-way valve is disconnected from the exhaust port. The pressure sensor outputs the monitored pressure to the second ventilation control apparatus. The second ventilation control apparatus determines whether the pressure exceeds the preset threshold, and outputs a corresponding control signal to the three-way valve, when determining that the pressure exceeds the preset threshold. When triggered by the control signal, the three-way valve disconnects the first port from the second port and connects(communicates) the first port with the exhaust port, thereby releasing the gas with excessive pressure and improving safety. The preset threshold can be set based on clinical experience or doctor requirements.

The human-machine interaction apparatus of the anesthesia machine also includes a display. A respiratory support mode can be selected by a user through the human-machine interaction apparatus. In this embodiment, one respiratory support mode corresponds to one ventilation mode, and selecting a ventilation mode is selecting a respiratory support mode. There are many specific human-machine interaction methods, and two of them are given below for illustration.

In a first human-machine interaction mode, the anesthesia machine displays a first ventilation mode and a second ventilation mode on a display interface (such as a main monitoring interface or a switching interface for ventilation mode) of a display for the user to select. Selecting the first ventilation mode means selecting a first respiratory support mode, which is to provide periodic respiratory support through the ventilation module. Selecting the second ventilation mode means selecting a second respiratory support mode, which is to provide anesthesia respiratory support for a patient. For example, an area for displaying ventilation state and an area for switching ventilation mode are provided on a same screen of the display interface of the display. The area for switching ventilation mode displays a first ventilation mode and a second ventilation mode for a user to select. After the user selects a respiratory support mode, the anesthesia machine provides corresponding respiratory support for a patient according to the selected respiratory support mode. The area for displaying ventilation state is configured to display preset ventilation state data when the anesthesia machine provides corresponding respiratory support for the patient according to the selected respiratory support mode. Wherein, the anesthesia machine can simultaneously display the first ventilation mode and the second ventilation mode in the area for switching ventilation mode for the user to select. Considering that a main function of the anesthesia machine is for anesthesia, the second ventilation mode can also be displayed in the area for switching ventilation mode while hiding the first ventilation mode, which can avoid the doctor from mistakenly selecting the first ventilation mode during anesthesia.

If the user selects the first ventilation mode through the human-machine interaction apparatus, that is, the respiratory support mode received by the human-machine interaction apparatus is the first respiratory support mode, the anesthesia machine displays a control window of the first respiratory support mode on the display interface for a user to set a ventilation parameter of the first ventilation mode; and then based on the ventilation parameter which is set by the user, the anesthesia machine enables the ventilation module to provide respiratory support for a patient, according to said ventilation parameter.

If the user selects the second ventilation mode through the human-machine interaction apparatus, that is, the respiratory support mode received by the human-machine interaction apparatus is the second respiratory support mode, the anesthesia machine displays a control window of the second respiratory support mode on the display interface for a user to set a ventilation parameter of the second ventilation mode; and then based on the ventilation parameter which is set by the user, the anesthesia machine provides anesthesia respiratory support for a patient .

Of course, the display interface can also allow a user to simultaneously select the first ventilation mode and the second ventilation mode (corresponding to a third respiratory support mode). After a third respiratory support mode is selected, the control window of the first respiratory support mode and the control window of the second respiratory support mode are displayed on the display interface for a user to enable the ventilation module to provide respiratory support for a patient, according to the ventilation parameter which is set by a user on the control window of the first respiratory support mode, and to enable the anesthesia machine to provide anesthesia respiratory support for another patient, according to the ventilation parameter which is set by a user on the control window of the second respiratory support mode.

In a second human-machine interaction mode, the anesthesia machine has at least two human-machine interaction apparatuses: a first human-machine interaction apparatus and a second human-machine interaction apparatus. The anesthesia machine displays a control window of a first ventilation mode on a display interface of the first human-machine interaction apparatus for a user to select the first ventilation mode and set a ventilation parameter of the first ventilation mode; displays a control window of the second ventilation mode on a display interface of the second human-machine interaction apparatus for a user to select the second ventilation mode and set a ventilation parameter of the second ventilation mode; after the first ventilation mode is selected, based on the ventilation parameter of the first ventilation mode which parameter is set by the user, the anesthesia machine enables the ventilation module to provide respiratory support for a patient according to said ventilation parameter; after the second ventilation mode is selected, based on the ventilation parameter of the second ventilation mode which parameter is set by the user, the anesthesia machine enables the first ventilation control apparatus to provide anesthesia respiratory support for a patient according to said ventilation parameter. The two ventilation modes are operated by two different human-machine interaction apparatuses, which can better prevent mistakes and avoid mis-operation, and also allow a user to focus more on the operation.

When the respiratory support mode, which is received by the human-machine interaction apparatus is to provide periodic respiratory support (first respiratory support mode), the display is configured to output information, which indicates to close the anesthetic delivery apparatus 20, or to switch an anesthesia mode for a patient, so as to avoid an anesthetic of the anesthetic delivery apparatus 20 being not provided to the patient through the respiratory loop but released into the environment. When the respiratory support mode received by the human-machine interaction apparatus is periodic respiratory support, the display can also be used to display the pressure waveform W (as shown in FIG. 9) for the convenience of user viewing and analysis.

In this embodiment, when the anesthetic delivery apparatus 20 is an electronic evaporation tank, the anesthesia machine is further configured to obtain a working state of the electronic evaporation tank. When the respiratory support mode, which is received by the human-machine interaction apparatus, is periodic respiratory support mode, the anesthesia machine is also configured to determine whether a current working state of the electronic evaporation tank is to stop working, that is, to determine whether the electronic evaporation tank is closed. Only after determining that the electronic evaporation tank is closed, the anesthesia machine is capable of switching to periodic respiratory support mode. Only after determining that the electronic evaporation tank is closed, the ventilation module 50 performs periodic respiratory support, which improves the safety of the anesthesia machine.

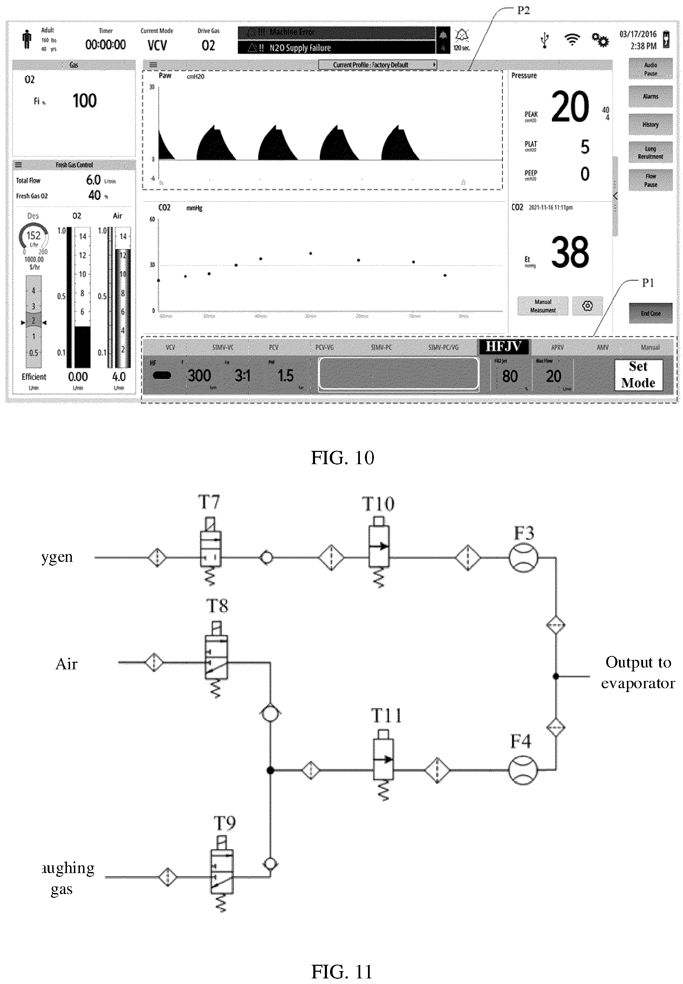

Further combined with FIG. 10, which is a diagram of a main monitoring interface on a display in an anesthesia machine provided by this disclosure. The main monitoring interface includes an area for switching ventilation mode P1. High-frequency jet ventilation (a first ventilation mode), as one of the ventilation modes, is presented in the area for switching ventilation mode P1 in a form of a tab. When it is desired to use the anesthesia machine to provide periodic respiratory support, an "HFJV" icon in the area for switching ventilation mode P1 is selected and a "SET MODE" icon is used to confirm, so as to complete the selection of periodic respiratory support mode. At this time, the ventilation module 50 can be controlled to start and provide periodic high-frequency jet ventilation to the patient. The pressure monitored during high-frequency jet ventilation is displayed in an area for displaying ventilation state (pressure waveform area) P2 of a main monitoring interface. When it is desired to use the anesthesia machine to provide periodic anesthesia respiratory support, an icon for another ventilation mode (second ventilation mode) in the area for switching ventilation mode P1, such as a "VCV" icon, can be selected. At this time, the first ventilation control apparatus 40 can control the respiratory loop to provide periodic anesthesia respiratory support (second respiratory support mode) for a patient.

In some embodiments, when the human-machine interaction apparatus receives any respiratory support mode, instructions based on the same respiratory support mode may be transmitted to the first ventilation control apparatus and the second ventilation control apparatus, respectively. The two ventilation control apparatuses respectively start or stop the corresponding respiratory support mode according to the received instructions. For example, when the respiratory support mode received by the human-machine interaction apparatus is periodic respiratory support, the first ventilation control apparatus 40 controls the respiratory loop to stop outputting the second gas with anesthetic to the outside, and stops providing periodic anesthesia respiratory support, for the patient, the second ventilation control apparatus synchronously controls the ventilation module 50 (for example, the gas delivery branch) to start periodically providing the first gas to the patient and perform high-frequency/jet ventilation.

In the above embodiments, the second ventilation control apparatus and the first ventilation control apparatus 40 are two independent ventilation control apparatuses. Of course, in other embodiments, the second ventilation control apparatus and the first ventilation control apparatus 40 may also be the same ventilation control apparatus, i.e., the control function of the ventilation module 50, may be further integrated with that of the first ventilation control apparatus. That is, the first ventilation control apparatus also has the function of the above-mentioned second ventilation control apparatus, and is configured to control the gas delivery branch to provide periodic respiratory support for a patient. In specific operations, after the human-machine interaction apparatus receives an instruction to select a respiratory support mode and transmits it to the first ventilation control apparatus, the first ventilation control apparatus determines whether to control the respiratory loop to provide periodic anesthesia respiratory support, and/or control the gas delivery branch to provide periodic respiratory support, to the patient.

As shown in FIGS. 6-8, the pressure sensor may be disposed between the second control valve T5 and the output port 530. The safety valve T6 is also provided between the second control valve T5 and the output port 530.

Multiple pressure sensors may be provided. **In** this embodiment, the first pressure sensor P1 and the second pressure sensor P2 are provided as an example for description. The pressure monitored by the first pressure sensor P1 is configured to trigger the safety valve T6, and the pressure monitored by the second pressure sensor P2 is configured to display the waveform.

Specifically, the first pressure sensor P1 is close to the safety valve T6. The first pressure sensor P1 monitors the pressure of the high-frequency gas. When it is found that the pressure value monitored by the first pressure sensor P1 is abnormal, the safety valve T6 is switched to separate the patient-side airway from the airway of the ventilation module 50. At the same time, the patient-side gas can be discharged through the safety valve T6, so as to prevent barotrauma at the patient side.

The second pressure sensor P2 is close to the patient end, that is, close to the output port 530. A pressure sampling port is provided at the patient end, which is connected with the second pressure sensor P2 of the ventilation module 50 through a pressure sampling tube. Therefore, the display displays, on the interface, a pressure oscillation waveform W, which is sampled by the second pressure sensor P2. Of course, in some embodiments, the action of the safety valve T6 can also be triggered based on the second pressure sensor P2, which is adjacent the patient end, and data of the pressure waveform W, which is displayed on the display, can also be sampled by the first pressure sensor P1, which is adjacent to the safety valve T6.

The ventilation module 50 can perform high-frequency/jet ventilation by controlling the switching frequency of the second control valve T5. The specific process of high-frequency/jet ventilation has been described in detail in the above content. The ventilation module 50 regulates the frequency of high-frequency/jet ventilation (i.e., the gas delivery frequency) of the second control valve T5 to achieve ventilation with different ventilation frequencies. For example, when the ventilation module 50 implements jet ventilation, the ventilation frequency of the jet ventilation apparatus can be lowered to form a low-frequency ventilation apparatus, and the low-frequency ventilation can be achieved with corresponding accessories (such as nasal plugs, nasal masks, masks, etc.) .

As shown in FIGS. 3 and 4, the anesthesia machine also includes a monitoring apparatus for flow amount 60. The monitoring apparatus for flow amount 60 is configured to control (regulate) the flow amount of the first gas. It can be mechanical or fully electronic. The monitoring apparatus for flow amount 60 can also be configured to detect the flow amount of the first gas. There are various connection methods between the monitoring apparatus for flow amount 60, the anesthetic delivery apparatus 20 and the ventilation module 50.

For example, in the embodiment shown in FIG. 3, the gas source module 10 is connected with the anesthetic delivery apparatus 20 through the monitoring apparatus for flow amount 60, wherein the ventilation module 50 is connected with the gas source module 10. In FIG. 3 specifically, the gas source interface is connected with the evaporator 210 through the monitoring apparatus for flow amount 60. In this way, the gas branch where the ventilation module 50 is located, and the anesthesia branch (such as the monitoring apparatus for flow amount 60, the anesthetic delivery apparatus 20 and the respiratory loop 30 in FIG. 3) share a first gas which is provided by the gas source module 10, so that the ventilation module 50 can be better integrated with the anesthesia machine in terms of gas path and control. Of course, in some embodiments, the anesthesia machine may also have two gas source modules 10: a first gas source module and a second gas source module; the structures and functions of the two gas source modules are described above. The second gas source module is connected with the anesthetic delivery apparatus 20 through the monitoring apparatus for flow amount 60 to provide a first gas for the anesthesia branch; the first gas source module is connected with the ventilation module 50 to provide a first gas for the ventilation module 50. In this embodiment, although the anesthesia branch and the ventilation module 50 both use a first gas, the gas paths are independent of each other. The first gas may also be humidified or otherwise processed before being delivered to the ventilation module 50. That is, the first gas described in this disclosure includes both a gas which is outputted by the gas source module, and a gas that has been humidified or otherwise processed at an output port of the gas source module.

For another example, in the embodiment shown in FIG. 4, the anesthesia machine also includes a three-way valve T12. The three-way valve T12 includes a first port 1 which is connected with the monitoring apparatus for flow amount 60, a second port 2 which is connected with the anesthetic delivery apparatus 20, and a third port 3 which is connected with the ventilation module 50. That is, the gas source module 10 is connected with an input end of the monitoring apparatus for flow amount 60. An output end of the monitoring apparatus for flow amount 60 can be connected with the anesthetic delivery apparatus 20 under the control of the three-way valve T12, or can be connected with the ventilation module 50 under the control of the three-way valve T12, that is, the three-way valve T12 is configured to enable the monitoring apparatus for flow amount 60 to connect with one of the anesthetic delivery apparatus 20 and the ventilation module 50.

The gas source module 10 may also include a laughing gas unit which provides a laughing gas. The laughing gas unit may be a laughing gas interface, which is connected with an external laughing gas source, such as a laughing gas pipeline in a hospital, a laughing gas bottle, etc. The monitoring apparatus for flow amount 60 may have three input interfaces, which are respectively connected with the oxygen interface, the air interface, and the laughing gas interface. FIG. 11 corresponds to the monitoring apparatus for flow amount 60 in the embodiment in FIG. 3. The monitoring apparatus for flow amount 60 includes a third switch valve T7, a fourth switch valve T8, a fifth switch valve T9, a fourth control valve T10, a fifth control valve T11, a third flow sensor F3 and a fourth flow sensor F4. One end of the third switch valve T7 is connected with the oxygen interface, and the other end of the third switch valve T7 is connected with the input end of the third flow sensor F3 through the fourth control valve T10. One end of the fourth switch valve T8 is connected with the air interface, and one end of the fifth switch valve T9 is connected with the laughing gas interface. The other end of the fourth switch valve T8 and the other end of the fifth switch valve T9 are converged with each other and then connected with the input end of the fourth flow sensor F4 through the fifth control valve T11. The output end of the third flow sensor F3 and the output end of the fourth flow sensor F4 are converged with each other and then connected with the evaporator 210. The third switch valve T7, the fourth switch valve T8 and the fifth switch valve T9 are all configured to control a connection/disconnection state of the gas path. The fourth control valve T10 and the fifth control valve T11 are used for flow amount control.

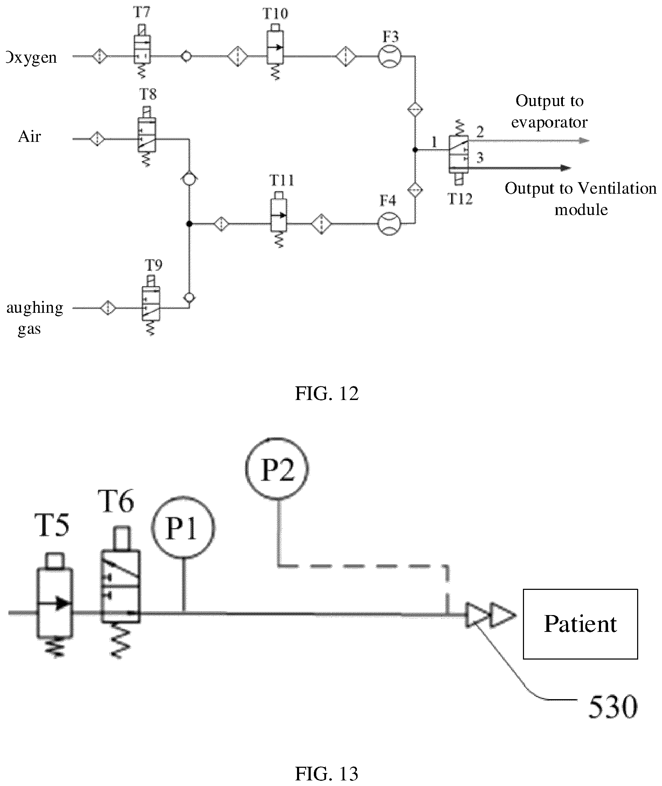

FIG. 12 corresponds to the monitoring apparatus for flow amount 60 of the embodiment in FIG. 4. The output end of the third flow sensor F3 and the output end of the fourth flow sensor F4 are converged with each other and then connected with the input port 1 of the three-way valve T12. The three-way valve T12 has a first output port 2 and a second output port 3. The first output port 2 of the three-way valve T12 is connected with the evaporator 210, and the second output port 3 of the three-way valve T12 is connected with the ventilation module (such as a high-frequency ventilation apparatus 501). The three-way valve T12 has two states, one state is that its input port 1 is connected with the first output port2, and the other state is that its input port 1 is connected with the second output port 3. That is, by controlling the three-way valve T12, it is possible to select whether the gas is outputted to the evaporator 210 or the high-frequency ventilation apparatus 501. The third switch valve T7, the fourth switch valve T8 and the fifth switch valve T9 are all configured to control a connection/disconnection state of the gas path. The fourth control valve T10 and the fifth control valve T11 are used for flow amount control.

In an embodiment shown in FIG. 4 , the ventilation module 50 may include a gas delivery branch and an output port, but the second control valve T5 is not required in the gas delivery branch. Instead, the gas delivery branch and the monitoring apparatus for flow amount 60 share the fourth control valve T10 and the fifth control valve T11, wherein the fourth control valve T10 and the fifth control valve T11 are all used for periodic ventilation control, that is, the functions of the fourth control valve T10 and the fifth control valve T11 can be the same as the above-mentioned second control valve. For example, both the fourth control valve T10 and the fifth control valve T11 can be controlled to switch an amount of flow at a certain frequency, so as to enable a pulse frequency of the gas flow at the first port 1 of the three-way valve T12 to be greater than or equal to 3Hz, or between 50-1500bpm. Specifically, taking FIG. 12 as an example, in an alternative embodiment, the first port 1 of the three-way valve T12 is connected with the monitoring apparatus for flow amount 60, the second port 2 of the three-way valve T12 is connected with the anesthetic delivery apparatus 20, the third port 3 of the three-way valve T12 is connected with the ventilation module 50. When the respiratory support mode received by the anesthesia machine is periodic respiratory support, the first port 1 and the third port 3 of the three-way valve T12 are connected, and the fourth control valve T10 and the fifth control valve T11 are connected with the ventilation module 50, so as to switch the flow amounts according to a preset frequency, thus enabling the ventilation module 50 to ventilate the patient at the first ventilation frequency. The first ventilation frequency may be, for example, greater than or equal to 50bpm, higher than or equal to 3 Hz, or between 50-1500 bpm. In this way, by switching opening degrees of the two control valves T10 and T11, the oxygen-air mixed gas (it can also be a mixed gas of oxygen, air and laughing gas) forms a periodic gas flow. This gas flow with high periodicity is transmitted along the gas delivery branch to the output port. If the output port 530 of the ventilation module 50 is connected with the jet accessory, the periodic gas flow passes through the jet accessory and then provides jet ventilation to the patient. When the respiratory support mode received by the anesthesia machine is periodic anesthesia respiratory support, the first port 1 and the second port 2 of the three-way valve T12 are connected, the fourth control valve T10 and the fifth control valve T11 are connected with the anesthetic delivery apparatus 20 for providing the first gas to the anesthetic delivery apparatus at a second ventilation frequency, so as to enable the first gas to be mixed with the anesthetic at the second ventilation frequency for obtaining the second gas and ventilating the patient by the respiratory loop. The first ventilation frequency is higher than the second ventilation frequency. The second ventilation frequency may be zero, that is, the fourth control valve T10 and the fifth control valve T11 can be opened.

It can be understood that in the embodiment shown in FIG. 4, gas can be introduced from a rear end of the monitoring apparatus for flow amount 60 to the ventilation module. Compared with the solution in FIG. 3, the ventilation module in the solution in FIG. 4 borrows (shares) some components (T10 and T11) of the monitoring apparatus for flow amount 60, which greatly simplifies the structure of the ventilation module.

It can be seen from the above that, the first gas is at least oxygen-containing gas, and the anesthesia machine may also include an auxiliary gas supply module, which is configured to receive the first gas, which is outputted by the gas source module 10, and to use the first gas to provide respiratory support for a patient at a flow amount of 20-80 litres/minute. Respiratory support provided by the auxiliary gas delivery module is non-periodic respiratory support. Non-periodic respiratory support refers to a continuous delivery of gas to the patient. This manner is actually oxygen therapy of high-flow amount. In this way, the anesthesia machine can provide the oxygen therapy of high-flow amount to patients when anesthesia is not needed, and has a wide range of applications.

In some embodiments, the anesthesia machine may include a main frame having a housing, and at least part of the ventilation module may be arranged inside an accommodation space formed by the housing, forming an integrated whole with the anesthesia machine. That is, at least part of the ventilation module is integrated inside the anesthesia machine and integrated with the anesthesia machine. For example, the second ventilation control apparatus, which is included in the ventilation module, is built into the main frame and is in communicative connection with the human-machine interaction apparatus of the anesthesia machine.

The anesthesia machine may also include a monitoring module for carbon dioxide. The monitoring module for carbon dioxide is configured to detect a fractional concentration of end-tidal carbon dioxide for a patient, when the first ventilation control apparatus provides anesthesia respiratory support for said patient, and then display said concentration on the display interface, and/or, configured to detect a fractional concentration of end-tidal carbon dioxide for a patient, when the ventilation module provides respiratory support for said patient, and then display said concentration on the display interface. An anesthesia machine usually has a monitoring module for carbon dioxide. This disclosure integrates a ventilation module into the anesthesia machine. When providing respiratory support for a patient, the ventilation module can use the monitoring module for carbon dioxide of the anesthesia machine to detect the fractional concentration of end-tidal carbon dioxide, such that the ventilation module does not need to have its own carbon dioxide sensor, which simplifies structure and improves integration.

The anesthesia machine of this disclosure, as integrated with a ventilation module, can provide following ventilation control: when the received respiratory support mode is periodic anesthesia respiratory support (a second respiratory support mode), controlling a first gas to mix with an anesthetic, so as to obtain a second gas, controlling the second gas to enter into a respiratory loop of the anesthesia machine, and controlling the respiratory loop to periodically output the second gas, so as to provide periodic anesthesia respiratory support; when the received respiratory support mode is periodic respiratory support (a first respiratory support mode), controlling a first gas to enter into a ventilation module of the anesthesia machine, and controlling the ventilation module to periodically output said first gas, so as to provide periodic respiratory support; wherein periodic respiratory support includes providing a gas delivery to a patient at a variable flow rate within one respiratory cycle (such as at two flow rates, one is large and the other one is small, during one respiratory cycle). For example, when the user wants to provide jet ventilation through the anesthesia machine, the ventilation module can be controlled to output periodic first gas at a certain frequency. The periodic first gas can complete jet ventilation after passing through the jet accessory.

This disclosure creatively integrates the jet Ventilation (JV) function into a general anesthesia machine, so as to support, by direct use of the anesthesia machine, applications, such as pharyngeal, tracheal, bronchial or lung surgeries where tracheal intubation ventilation is not applicable. For example, when using the anesthesia machine provided by this disclosure, the doctor puts the jet needle or nozzle into a suitable position in the pharynx or trachea, and uses the anesthesia machine to perform jet ventilation and respiratory support. On the one hand, the diameter of the jet needle or nozzle is very small, leaving enough space for the doctor to observe and perform laryngoscopy surgery. On the other hand, due to the characteristics of jet ventilation, especially the characteristics of high-frequency (of 50bpm or above, of 3Hz or above, or 50-1500bpm) jet ventilation, a pulmonary pressure of the patient changes very little between inhalation and exhalation, so that no large fluctuations are caused in the pulmonary respiration, which undoubtedly provides convenience for one-lung ventilation or a pulmonary surgery. It is easy to understand that because jet ventilation uses a jet needle and a nozzle for ventilation, no sealed connection is formed between the airway system of the anesthesia machine and the respiratory system of the patient. Therefore, this type of ventilation cannot simultaneously inject aesthetics through the jet needle and nozzle. Otherwise, the anesthetic may leak out. In this case, the anesthesia needs to be administered intravenously.

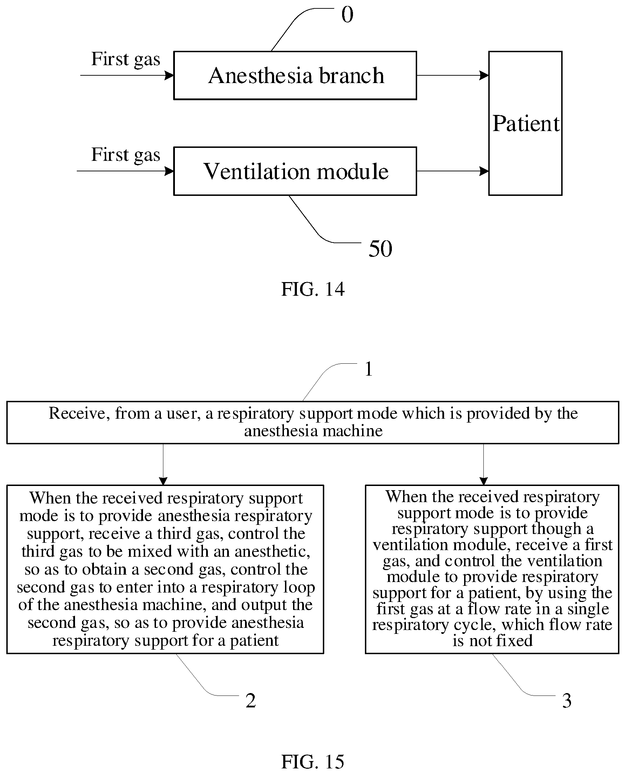

As shown in FIG. 14, this disclosure further provides an anesthesia machine, including: an anesthesia branch 0, a ventilation module 50 and a housing. The anesthesia branch 0 is mainly used for anesthesia, and the ventilation module 50 is mainly used for ventilation. Output ports of the anesthesia branch 0 and the ventilation module 50 are different, and output gases of the anesthesia branch 0 and the ventilation module 50 are also different. The anesthesia branch 0 is configured to receive a first gas, mix the first gas with an anesthetic to obtain a second gas, and use the second gas to provide anesthesia respiratory support for a patient at a third ventilation frequency. The anesthesia branch 0 specifically delivers the second gas to said patient through its output port. The ventilation module 50 is configured to receive a first gas and provide respiratory support for a patient, by using said first gas at a first ventilation frequency. The ventilation module 50 includes: a gas delivery branch and an output port. The gas delivery branch is configured to receive a first gas. At least a portion of the ventilation module 50 is arranged inside an accommodation space which is formed by the housing, wherein said portion of the ventilation module and the anesthesia machine form a whole

The ventilation module 50 may be a ventilation module 50 in the above-mentioned embodiment. The ventilation module 50 may further include a second control valve, which is configured to enable the first gas inside the gas delivery branch to form a gas flow at a first ventilation frequency, which may referring the above embodiments for details, and is not described here in detail. Of course, two control valves may be used to replace the second control valve. That is, in some embodiments, the ventilation module 50 may further include a first control valve and a third control valve, wherein the first control valve and the third control valve together enable the first gas in the gas delivery branch to form a gas flow at a first ventilation frequency, which may referring the above embodiments for details, and is not described here in detail.

The anesthesia branch 0 may include an anesthetic delivery apparatus 20 and a respiratory loop 30 in the above embodiment, and may further include a monitoring apparatus for flow amount 60, etc. For details, please refer to above embodiments, which are not described here. The anesthesia machine may further include a gas source module 10. The anesthesia branch 0 can share the gas source module 10 with the ventilation module 50, such as sharing the above-mentioned gas source interfaces, that is, the anesthesia branch 0 and the ventilation module 50 are connected with each other, or both connected with the gas source interface together. Of course, the anesthesia branch 0 and the ventilation module 50 can also be connected with different gas source modules 10 respectively, so that the gas paths are not connected.

This disclosure also provides an anesthesia machine, including: a monitoring apparatus for flow amount, a respiratory loop, a first ventilation control apparatus and a human-machine interaction apparatus. The monitoring apparatus for flow amount is configured to adjust a flow amount of a third gas; wherein after the flow amount of the third gas is regulated by the monitoring apparatus for flow amount, the third gas is delivered to an anesthetic delivery apparatus and then mixed with an anesthetic, so as to obtain a second gas. Said monitoring apparatus for flow amount is same as the monitoring apparatus for flow amount of the above embodiment except that it adjusts the flow amount of the third gas instead of the flow amount of the first gas, so it is not described here in detail. Likewise, said anesthetic delivery apparatus is the same as the above embodiment, except that the first gas is changed to the third gas. The respiratory loop is configured to receive the second gas and deliver the second gas to a patient. The function of said respiratory loop is same as that of the respiratory loop in the above embodiment, and is not described in detail here.

The first ventilation control apparatus is configured to control the respiratory loop to deliver the second gas to a patient, so as to provide anesthesia respiratory support for said patient. Likewise, the function of said first ventilation control apparatus is same as that of the first ventilation control apparatus in the above embodiment, and is not described in detail herein. The human-machine interaction apparatus is configured to receive, from a user, a respiratory support mode for a patient which is provided by the anesthesia machine. Similarly, the function of said human-machine interaction apparatus is same as that of the human-machine interaction apparatus in the above embodiment, and is not described in detail here.

The anesthesia machine further includes a ventilation module. The ventilation module is configured to receive a first gas and provide respiratory support for a patient of multiple respiratory cycles, by using said first gas at a first ventilation frequency; wherein the ventilation module is configured to deliver a first gas to a patient at a variable flow rate within a single respiratory cycle. Likewise, the function of said ventilation module is same as that of the ventilation module in the above embodiment, and is not described in detail here.

As shown in FIG. 15, a process of ventilation control by an anesthesia machine may include following steps.

Step 1, the anesthesia machine receives, from a user, a respiratory support mode, which is provided by the anesthesia machine through a human-machine interaction apparatus.

Step 2, when the received respiratory support mode is to provide anesthesia respiratory support, the monitoring apparatus for flow amount receives a third gas which is provided by a gas source module, controls the third gas to be mixed with an anesthetic, so as to obtain a second gas, for example, delivering the third gas whose flow amount has been regulated by the monitoring apparatus for flow amount to an anesthetic delivery apparatus, and mixing the third gas with an anesthetic in the anesthetic delivery apparatus to obtain the second gas, and then a first ventilation control apparatus controls the second gas to enter into a respiratory loop of the anesthesia machine, and controls the respiratory loop to output the second gas, so as to provide anesthesia respiratory support for a patient.

Step 3, when the received respiratory support mode is to provide respiratory support through a ventilation module, the ventilation module receives a first gas, and provides respiratory support for a patient, by using the first gas at a flow rate in a single respiratory cycle, which flow rate is not fixed. Of course, there is another respiratory support mode in which steps 2 and 3 are simultaneously performed. The specific process is shown in the above embodiment and is not described in detail here.

The third gas may be same as the first gas, and the third gas and the first gas may come from a same gas source module or from different gas source modules. The specific solution is in the above embodiment. **In** this embodiment, the third gas is different from the first gas, and components of the third gas and the first gas are different. At least portion of the gases of the third gas and the first gas are from different gas sources. For example, the third gas contains nitrous oxide, while the first gas does not contain nitrous oxide. In this embodiment, the third gas and the first gas come from different gas source modules, and gas paths of the third gas and the first gas are not connected.

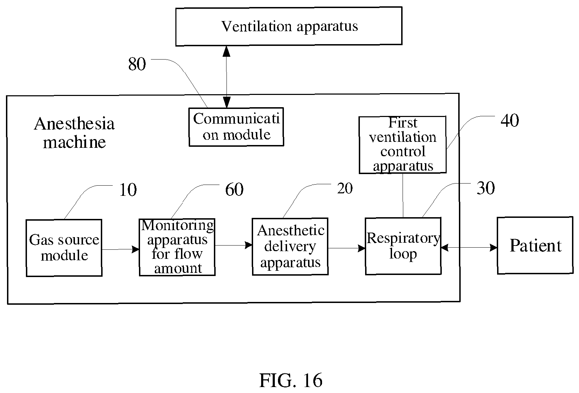

The above-mentioned anesthesia machines are all integrated with a ventilation module. Of course, the ventilation module may not be integrated into the anesthesia machine, but may be remotely controlled by the anesthesia machine. For example, as shown in FIG. 16, an anesthesia machine includes: a gas source module 10, a respiratory loop 30, a first ventilation control apparatus 40, a human-machine interaction apparatus, and a communication module 80.

The gas source module 10 is connected with an anesthetic delivery apparatus. For example, the gas source module 10 includes a gas source interface. One end of the gas source interface is connected with a gas source, and the other end of the gas source interface is connected with an anesthetic delivery apparatus 20, and specifically can be connected with the anesthetic delivery apparatus 20 through a monitoring apparatus for flow amount 60. The anesthetic delivery apparatus 20 is configured to mix a third gas, which is provided by the gas source, with an anesthetic, and deliver to the respiratory loop 30 a second gas which is obtained after the mixing.

The respiratory loop 30 is configured to deliver the second gas to a patient. The first ventilation control apparatus 40 is configured to control the respiratory loop 30 to deliver the second gas to the patient, thereby providing anesthesia respiratory support for the patient. The human-machine interaction apparatus is configured to receive, from a user, a respiratory support mode which is provided by the anesthesia machine to the patient. The functions of the gas source module 10, the monitoring apparatus for flow amount 60, the anesthetic delivery apparatus 20, the respiratory loop 30, the first ventilation control apparatus 40 and the human-machine interaction apparatus are the same as those in the above embodiment and are not described in detail here.

The communication module 80 is configured to communicate with an external ventilation apparatus, either by wired communication or wireless communication. The external ventilation apparatus is configured to provide respiratory support for a patient, by using a first gas at a preset first ventilation frequency; the first ventilation frequency is not zero. That is, the function of the ventilation apparatus of this embodiment is the same as that of the ventilation module of the above-mentioned embodiment, except that the ventilation apparatus is independent of the anesthesia machine. The ventilation apparatus may specifically be a high-frequency ventilation apparatus (e.g., a high-frequency jet ventilation apparatus), a low-frequency ventilation apparatus, etc. The specific functions thereof are shown in the ventilation module in the above embodiment and are not described in detail here.

The anesthesia machine may also be configured to, according to a respiratory support mode received by the human-machine interaction apparatus, provide anesthesia respiratory support for a patient, and/or control the ventilation apparatus through the communication module 80, so as to enable the ventilation apparatus to provide respiratory support for a patient. In an embodiment in which the ventilation module is built into a housing of the anesthesia machine, the anesthesia machine directly controls the internal ventilation module to provide respiratory support for the patient. However, in this embodiment, the anesthesia machine controls the external ventilation apparatus through the communication module 80 to provide respiratory support for a patient. Apart from this, the ventilation module and the ventilation apparatus have the same function, that is, both use the first gas at the first ventilation frequency to provide respiratory support for the patient in the same way.

Similarly, the anesthesia machine can have multiple ventilation modes, and there can be multiple human-machine interaction apparatuses for selecting ventilation modes. Three of them are cited below for illustration.

In a first human-machine interaction mode, the anesthesia machine displays a first ventilation mode and a second ventilation mode on a display interface (such as a main monitoring interface or a switching interface for ventilation mode) of a display at a human-machine interaction apparatus for a user to select. Selecting the first ventilation mode means selecting a first respiratory support mode, which is to provide respiratory support through the ventilation apparatus. Selecting the second ventilation mode means selecting a second respiratory support mode, which is to provide anesthesia respiratory support for a patient. For example, an area for displaying ventilation state and an area for switching ventilation mode are provided on a same screen of the display interface of the display. The area for switching ventilation mode displays a first ventilation mode and a second ventilation mode for a user to select. Wherein, the anesthesia machine can simultaneously display the first ventilation mode and the second ventilation mode in the area for switching ventilation mode for a user to select. Considering that a main function of the anesthesia machine is for anesthesia, the second ventilation mode can also be displayed in the area for switching ventilation mode while hiding the first ventilation mode, which can avoid the doctor from mistakenly selecting the first ventilation mode during anesthesia.

If the user selects the first ventilation mode through the human-machine interaction apparatus, that is, the respiratory support mode received by the human-machine interaction apparatus is the first respiratory support mode, the anesthesia machine displays a control window of the first respiratory support mode in the area for switching ventilation mode on the display interface for a user to set a ventilation parameter of the first ventilation mode; and then based on the ventilation parameter which is set by the user, the anesthesia machine control, through the communication module 80, the ventilation apparatus to provide respiratory support for a patient, according to said ventilation parameter.

If the user selects the second ventilation mode through the human-machine interaction apparatus, that is, the respiratory support mode received by the human-machine interaction apparatus is the second respiratory support mode, the anesthesia machine displays a control window of the second respiratory support mode in the area for switching ventilation mode on the display interface for a user to set a ventilation parameter of the second ventilation mode; and then based on the ventilation parameter which is set by the user, the anesthesia machine provides anesthesia respiratory support for a patient .

Of course, the display interface can also allow a user to simultaneously select the first ventilation mode and the second ventilation mode (corresponding to a third respiratory support mode). After a third respiratory support mode is selected, the control window of the first respiratory support mode and the control window of the second respiratory support mode are displayed on the display interface for a user, so as to control, through the communication module 80, the ventilation apparatus to provide respiratory support for a patient, according to a ventilation parameter which is set by a user on the control window of the first respiratory support mode, and to enable the anesthesia machine to provide anesthesia respiratory support for another patient, according to a ventilation parameter which is set by a user on the control window of the second respiratory support mode.

In the first human-computer interaction mode, the anesthesia machine displays the area for displaying ventilation state (i.e., pressure waveform area) and the area for switching ventilation mode on the same main monitoring interface, providing a flat layout design for switching ventilation mode. In this interactive mode, the first respiratory support mode and the second respiratory support mode are both integrated in the area for switching a ventilation mode. For a user of the anesthesia machine, remote control of the ventilation apparatus becomes a special ventilation mode, which can better adapt to a function expansion of anesthesia machine without changing a daily operating habit for switching a ventilation mode. For the main monitoring interface, although an alternative respiratory support mode has been added, a display area of the area for displaying ventilation state has not been compressed.

In a second human-machine interaction mode, the anesthesia machine has at least two human-machine interaction apparatus: a first human-machine interaction apparatus and a second human-machine interaction apparatus. The anesthesia machine displays a control window of a first ventilation mode on a display interface of the first human-machine interaction apparatus for a user to select the first ventilation mode and set a ventilation parameter of the first ventilation mode; displays a control window of a second ventilation mode on a display interface of the second human-machine interaction apparatus for a user to select the second ventilation mode and set a ventilation parameter of the second ventilation mode; after the first ventilation mode is selected, based on the ventilation parameter of the first ventilation mode which parameter is set by the user, the anesthesia machine controls, through the communication module 80, the ventilation apparatus to provide respiratory support for a patient according to said ventilation parameter; after the second ventilation mode is selected, based on the ventilation parameter of the second ventilation mode which parameter is set by the user, the anesthesia machine provides anesthesia respiratory support for a patient. The two ventilation modes are operated by two different human-machine interaction apparatuses, which can better prevent mistakes and avoid mis-operation, and also allow a user to focus more on the operation.

Considering that the anesthesia machine does not necessarily need to control the ventilation apparatus every time, when the anesthesia machine is used, the anesthesia machine can display the first ventilation mode on the display interface of the first human-machine interaction apparatus only after the anesthesia machine is in communicative connection with the ventilation apparatus through the communication module 80, so as to enable a user to activate the control window of the first respiratory support mode. That is to say, after the anesthesia machine is in communicative connection with the ventilation apparatus, the anesthesia machine can be controlled to display a control window of the first respiratory support mode on one of its displays, thereby allowing the user to control the ventilation apparatus, which is very convenient.

In a third human-computer interaction mode, during a daily usage of the anesthesia machine, an operation interface of the anesthesia machine is displayed on the display interface of its human-machine interaction apparatus, so that the user can perform routine operations on the anesthesia machine. For example, the operation interface of the anesthesia machine is for a user to select a second respiratory support mode and set a ventilation parameter of the second respiratory support mode. After the second respiratory support mode is selected, the anesthesia machine enables the first ventilation control apparatus to provide anesthesia respiratory support for a patient.

The display interface of the human-machine interaction apparatus of the anesthesia machine also displays an operation interface of the ventilation apparatus, so that the user can directly operate the ventilation apparatus through said operation interface. For example, the operation interface of the ventilation apparatus is for a user to select a first respiratory support mode and set a ventilation parameter of the first respiratory support mode. After the first respiratory support mode is selected, the anesthesia machine controls, through the communication module 80, the ventilation apparatus to provide respiratory support for a patient.

Similarly, the anesthesia machine may display, on an operation interface of the anesthesia machine, an operation interface of the ventilation apparatus, only after the anesthesia machine is in communicative connection with the ventilation apparatus through the communication module 80.

Since the difference between the embodiment shown in FIG. 16 and the embodiment shown in FIGS. 2~15 lies in a remote control of the ventilation apparatus, and the ventilation apparatus provides respiratory support for the patient in the same way as that the aforementioned ventilation module provides respiratory support for the patient, the details are shown in the aforementioned embodiments and are not repeated here.

This disclosure integrates ventilation modules, such as jet ventilation functions, into a general anesthesia machine, and integrates board cards, display screens, etc. into one device, which brings convenience to the management of surgery device and allows more hospitals to perform pharyngeal surgery. It has great clinical value for anesthesia surgeries that cannot be performed with tracheal intubation and ventilation, such as tracheal, bronchial or lung surgeries.

The description has been made with reference to various exemplary embodiments herein. However, those skilled in the art recognize that changes and modifications can be made to the exemplary embodiments without departing from the scope herein. For example, various operation steps and components for performing operation steps may be implemented in different ways according to a specific application or considering any number of cost functions associated with the operation of the system (for example, one or more steps may be deleted, modified, or incorporated into other steps).

In addition, as understood by those skilled in the art, the principles herein may be reflected in a computer program product on a computer-readable storage medium that is pre-installed with computer-readable program codes. Any tangible, non-transitory computer-readable storage medium can be used, including magnetic storage devices (hard disks, floppy disks, etc.), optical storage devices (CD-ROM, DVD, Blu Ray disks, etc.), flash memory, and/or the like. These computer program instructions can be loaded onto a general-purpose computer, a dedicated computer, or other programmable data processing device to form a machine, so that these instructions executed on a computer or other programmable data processing apparatus can generate an apparatus that implements a specified function. These computer program instructions can also be stored in a computer-readable memory that can instruct a computer or other programmable data processing device to operate in a specific manner, so that the instructions stored in the computer-readable memory can form a manufactured product, including an implementation apparatus that implements a specified function. The computer program instructions can also be loaded onto a computer or other programmable data processing device, so that a series of operating steps are performed on the computer or other programmable device to produce a computer-implemented process, so that the instructions executed on a computer or other programmable data processing device can provide steps for implementing specified functions.

Although the principles herein have been shown in various embodiments, many modifications of structures, arrangements, proportions, elements, materials, and components particularly suitable for the specific environmental and operational requirements may be used without departing from the principles and scope of the present disclosure. The above modifications and other changes or amendments are included in the scope of this disclosure.

The foregoing specific description has been described with reference to various embodiments. However, those skilled in the art recognize that various modifications and changes can be made without departing from the scope of the present disclosure. Therefore, consideration of the present disclosure is in an illustrative rather than a restrictive sense, and all such modifications are included within the scope thereof. Also, the advantages of various embodiments, other advantages, and the solutions to problems have been described above. However, the benefits, advantages, solutions to problems, and any elements that can produce these, or solutions that make them more explicit, should not be interpreted as critical, necessary, or essential. The term "comprising", and any other variants thereof used herein are non-exclusive, so that the process, method, document, or device that includes a list of elements includes not only these elements, but also other elements that are not explicitly listed or do not belong to the process, method, system, document, or device. Furthermore, the term "coupling" and any other variations thereof used herein refer to physical connection, electrical connection, magnetic connection, optical connection, communicational connection, functional connection, and/or any other connection.

It should be noted that a person of ordinary skill in the art could also make several variations and improvements without departing from the concept of this disclosure, and these variations and improvements would all fall within the scope of protection of this disclosure. Therefore, the protection scope of this disclosure should be in accordance with the appended claims.

## Claims

1. An anesthesia machine, comprising a housing, a monitoring apparatus for flow amount, a respiratory loop, a first ventilation control apparatus and a human-machine interaction apparatus;
the monitoring apparatus for flow amount is configured to regulate a flow amount of a first gas; wherein after the flow amount of said first gas is regulated by the monitoring apparatus for flow amount, said first gas is delivered to an anesthetic delivery apparatus and then mixed with an anesthetic which is provided by the anesthetic delivery apparatus, so as to obtain a second gas;
the respiratory loop is configured to receive the second gas and deliver the second gas to a patient;
the first ventilation control apparatus is configured to control the respiratory loop to deliver the second gas to said patient, so as to provide anesthesia respiratory support for said patient;
the human-machine interaction apparatus is configured to receive, from a user, a respiratory support mode for a patient which is provided by the anesthesia machine;
**characterized in that**, the anesthesia machine further comprises:
a ventilation module, which is configured to receive a first gas and provide respiratory support for a patient, by using said first gas at a first ventilation frequency; wherein at least a portion of the ventilation module is arranged inside an accommodation space which is formed by the housing, wherein said portion of the ventilation module and the anesthesia machine form a whole;
the anesthesia machine is further configured to enable the anesthesia machine to provide the anesthesia respiratory support for a patient and/or provide the respiratory support for a patient, according to the respiratory support mode which is received by the human-machine interaction apparatus.

2. An anesthesia machine, comprising a monitoring apparatus for flow amount, a respiratory loop, a first ventilation control apparatus and a human-machine interaction apparatus;
the monitoring apparatus for flow amount is configured to regulate a flow amount of a third gas; wherein after the flow amount of the third gas is regulated by the monitoring apparatus for flow amount, the third gas is delivered to an anesthetic delivery apparatus and then mixed with an anesthetic, so as to obtain a second gas;
the respiratory loop is configured to receive the second gas and deliver the second gas to a patient;
the first ventilation control apparatus is configured to control the respiratory loop to deliver the second gas to said patient, so as to provide anesthesia respiratory support for said patient;
the human-machine interaction apparatus is configured to receive, from a user, a respiratory support mode for a patient which is provided by the anesthesia machine;
**characterized in that**, the anesthesia machine further comprises:
a ventilation module, which is configured to receive a first gas and provide respiratory support for a patient of multiple respiratory cycles, by using said first gas at a first ventilation frequency; wherein the ventilation module is configured to deliver said first gas to a patient at a variable flow rate within a single respiratory cycle;
the anesthesia machine is further configured to enable the anesthesia machine to provide the anesthesia respiratory support for a patient and/or provide the respiratory support for a patient, according to the respiratory support mode which is received by the human-machine interaction apparatus.

3. An anesthesia machine, **characterized in that**, comprising:
an anesthesia branch, which is configured to receive a first gas, mix said first gas with an anesthetic to obtain a second gas, and use the second gas to provide anesthesia respiratory support for a patient at a third ventilation frequency;
a ventilation module, which is configured to receive a first gas and provide respiratory support for a patient, by using said first gas at a first ventilation frequency; wherein the ventilation module comprises a gas delivery branch and an output port, the gas delivery branch is configured to receive said first gas; wherein the ventilation module further comprises a second control valve, which is configured to enable said first gas inside the gas delivery branch to form a gas flow at the first ventilation frequency; or, the ventilation module further comprises a first control valve and a third control valve, which together enable said first gas inside the gas delivery branch to form a gas flow at the first ventilation frequency; the output port is configured to output the gas flow at the first ventilation frequency; and
a housing, wherein at least a portion of the ventilation module is arranged inside an accommodation space which is formed by the housing, wherein said portion of the ventilation module and the anesthesia machine form a whole.

4. The anesthesia machine according to claim 3, **characterized in that**, the first ventilation frequency and the third ventilation frequency are both greater than zero.

5. The anesthesia machine according to any one of claims 1-3, **characterized in that**, the first ventilation frequency is not less than 50 times/minute; the ventilation module comprises a high-frequency ventilation apparatus, which is configured to ventilate a patient at a ventilation frequency of at least 50 times/minute, so as to provide the respiratory support for said patient.

6. The anesthesia machine according to claim 5, **characterized in that**, the high-frequency ventilation apparatus comprises a gas delivery branch, a second control valve and an output port; wherein the high-frequency ventilation apparatus is configured to receive, through the gas delivery branch, said first gas; the second control valve is arranged inside the gas delivery branch, and is controlled to switch an amount of flow at a switching frequency of at least 50 times/minute, so as to enable said first gas, which is outputted from the output port, to form a high-frequency gas flow.

7. The anesthesia machine according to claim 5, **characterized in that**, further comprising a jet accessory, which is connected with the high-frequency ventilation apparatus, wherein the jet accessory is configured to receive said first gas, which is outputted by the high-frequency ventilation apparatus, and enable said first gas to be outputted in a gas flow of high-frequency jet.

8. The anesthesia machine according to any one of claims 1-7, **characterized in that**, further comprising a gas source module which provides the first gas, wherein the gas source module comprises an oxygen unit, and the first gas comprises oxygen, which is provided by the oxygen unit;
the ventilation module comprises an oxygen branch, a second control valve and an output port; wherein the oxygen unit is connected with the output port through the oxygen branch, the second control valve is arranged inside the oxygen branch, so as to control a flow amount of oxygen inside the oxygen branch and enable said oxygen, which is outputted by the output port, to form a high-frequency gas flow.

9. The anesthesia machine according to any one of claims 1-7, **characterized in that**, further comprising a gas source module which provides the first gas, wherein the gas source module comprises an air unit, and the first gas comprises air, which is provided by the air unit;
the ventilation module comprises an air branch, a second control valve and an output port; wherein the air unit is connected with the output port through the air branch, the second control valve is arranged inside the air branch, so as to control a flow amount of air inside the air branch and enable said air, which is outputted by the output port, to form a high-frequency gas flow.

10. The anesthesia machine according to any one of claims 1-7, **characterized in that**, further comprising a gas source module which provides the first gas, wherein the gas source module comprises an oxygen unit which is configured to provide oxygen, and an air unit which is configured to provide air; the first gas comprises: the oxygen which is provided by the oxygen unit, and the air which is provided by the air unit;
the ventilation module comprises an oxygen branch, an air branch and an output port; wherein the oxygen branch is connected with the oxygen unit, the air branch is connected with the air unit; the oxygen branch and the air branch are converged with each other and then connected with the output port; wherein a first control valve is arranged inside the oxygen branch, so as to control a flow amount of oxygen inside the oxygen branch and enable said oxygen inside the oxygen branch to form a high-frequency gas flow; a third control valve is arranged inside the air branch, so as to control a flow amount of air inside the air branch and enable said air inside the air branch to form a high-frequency gas flow.

11. The anesthesia machine according to any one of claims 1-7, **characterized in that**, further comprising a gas source module which provides the first gas, wherein the gas source module comprises an oxygen unit which is configured to provide oxygen, and an air unit which is configured to provide air; the first gas comprises: the oxygen which is provided by the oxygen unit, and the air which is provided by the air unit;
the ventilation module comprises an oxygen branch, an air branch, a second control valve and an output port; wherein the oxygen branch is connected with the oxygen unit, the air branch is connected with the air unit, the oxygen branch and the air branch are converged with each other and then connected with the output port through the second control valve; wherein a first control valve is arranged inside the oxygen branch, a third control valve is arranged inside the air branch, wherein the first control valve is configured to control a flow amount of oxygen inside the oxygen branch, the third control valve is configured to control a flow amount of air inside the air branch, the second control valve is configured to enable a mixed gas, which is formed after converging said air and said oxygen, to form a high-frequency gas flow.

12. The anesthesia machine according to any one of claims 8-11, **characterized in that**, a ventilation frequency of the high-frequency gas flow, which flow is formed at the output port for ventilating a patient, is at least 50 times/minute.

13. The anesthesia machine according to any one of claims 8-11, **characterized in that**, the output port is connected with a jet accessory, and the high-frequency gas flow, which is outputted by the output port, forms a gas flow of high-frequency jet after passing through the jet accessory.

14. The anesthesia machine according to claim 13, **characterized in that**, a ventilation frequency of the gas flow of high-frequency jet for ventilating a patient is 50-1500 bpm.

15. The anesthesia machine according to any one of claims 8-9, or 11, **characterized in that**, a pressure sensor, which is configured to monitor a pressure, is provided between the second control valve and the output port;
the human-machine interaction apparatus comprises a display; wherein the display is configured to display waveform(s) of the pressure, which pressure is monitored by the pressure sensor, when a respiratory support mode, which is received by the human-machine interaction apparatus, is to provide respiratory support through the ventilation module.

16. The anesthesia machine according to claim 15, **characterized in that**, a safety valve, which is configured to release the pressure, is further provided between the second control valve and the output port.

17. The anesthesia machine according to any one of claims 1-3, **characterized in that**, the ventilation module comprises a gas delivery branch, a second control valve and an output port; wherein the second control valve is arranged inside the gas delivery branch, so as to control the ventilation module to provide respiratory support which is periodic, the ventilation module is further configured to receive, through the gas delivery branch, said first gas; wherein said first gas is outputted through a jet accessory, which is connected with the output port, so as to form a jet gas flow.

18. The anesthesia machine according to claim 1, **characterized in that**, further comprising a jet accessory, which is connected with the ventilation module, wherein the jet accessory is configured to receive said first gas, which is outputted by the ventilation module, and to enable said first gas to form a jet gas flow.

19. The anesthesia machine according to claim 18, **characterized in that**, the jet accessory comprises an jet channel, wherein an output aperture of the jet channel is less than 4 mm.

20. The anesthesia machine according to any one of claims 1-3, **characterized in that**, further comprising a gas source module which provides the first gas, wherein the gas source module comprises an oxygen unit, and the first gas comprises oxygen, which is provided by the oxygen unit;
the ventilation module comprises an oxygen branch, a second control valve and an output port; wherein the oxygen unit is connected with the output port through the oxygen branch, the output port is connected with a jet accessory, the second control valve is arranged inside the oxygen branch, so as to control a flow amount of oxygen inside the oxygen branch and enable said oxygen, which is outputted by the output port, to form a periodic jet flow after passing through the jet accessory.

21. The anesthesia machine according to claims 1-3, **characterized in that**, further comprising a gas source module which provides the first gas, wherein the gas source module comprises an air unit, and the first gas comprises air, which is provided by the air unit;
the ventilation module comprises an air branch, a second control valve and an output port; wherein the air unit is connected with the output port through the air branch, the output port is connected with a jet accessory, the second control valve is arranged inside the air branch, so as to control a flow amount of air inside the air branch and enable said air, which is outputted by the output port, to form a periodic jet flow after passing through the jet accessory.

22. The anesthesia machine according to claims 1-3, **characterized in that**, further comprising a gas source module which provides the first gas, wherein the gas source module comprises an oxygen unit which is configured to provide oxygen, and an air unit which is configured to provide air; the first gas comprises: the oxygen which is provided by the oxygen unit, and the air which is provided by the air unit;
the ventilation module comprises an oxygen branch, an air branch and an output port; wherein the oxygen branch is connected with the oxygen unit, the air branch is connected with the air unit; the oxygen branch and the air branch are converged with each other and then connected with the output port, wherein the output port is further connected with a jet accessory; wherein a first control valve is arranged inside the oxygen branch, so as to control a flow amount of oxygen inside the oxygen branch and enable said oxygen inside the oxygen branch to form a periodic gas flow; a third control valve is arranged inside the air branch, so as to control a flow amount of air inside the air branch and enable said air inside the air branch to form a periodic gas flow;
wherein the jet accessory is connected with the output port and configured to form a periodic jet gas flow, when a mixed gas, which is formed after converging the periodic gas flow of the oxygen branch and the periodic gas flow of the air branch.

23. The anesthesia machine according to claims 1-3, **characterized in that**, further comprising a gas source module which provides the first gas, wherein the gas source module comprises an oxygen unit which is configured to provide oxygen, and an air unit which is configured to provide air; the first gas comprises: the oxygen which is provided by the oxygen unit, and the air which is provided by the air unit;
the ventilation module comprises an oxygen branch, an air branch, a second control valve and an output port; wherein the oxygen branch is connected with the oxygen unit, the air branch is connected with the air unit, the oxygen branch and the air branch are converged with each other and then connected with the output port through the second control valve; wherein the output port is further connected with a jet accessory; wherein a first control valve is arranged inside the oxygen branch, a third control valve is arranged inside the air branch, wherein the first control valve is configured to control a flow amount of oxygen inside the oxygen branch, the third control valve is configured to control a flow amount of air inside the air branch, the second control valve is configured to enable a mixed gas, which is formed after converging said air and said oxygen, to form a periodic jet gas after passing through the jet accessory, which is connected with the output port.

24. The anesthesia machine according to any one of claims 1-3, **characterized in that**, the first ventilation frequency is greater than or equal to 10bpm, less than 3Hz, or greater than 20Hz.

25. The anesthesia machine according to claim 1 or claim 2, **characterized in that**, the ventilation module comprises a second ventilation control apparatus and a gas delivery branch; wherein the second ventilation control apparatus is configured to control the gas delivery branch to provide the respiratory support for a patient.

26. The anesthesia machine according to claim 1 or claim 2, **characterized in that**, the ventilation module comprises a gas delivery branch, wherein the first ventilation control apparatus is further configured to control the gas delivery branch to provide the respiratory support for a patient.

27. The anesthesia machine according to claim 1 or claim 2, **characterized in that**, the human-machine interaction apparatus comprises a display; wherein the display is configured to output information, which indicates to close the anesthetic delivery apparatus or to switch an anesthesia mode for a patient, when the respiratory support mode, which is received by the human-machine interaction apparatus, is to provide respiratory support through the ventilation module.

28. The anesthesia machine according to claim 1 or claim 2, **characterized in that**, the anesthetic delivery apparatus is an electronic evaporation tank;
wherein the anesthesia machine is further configured to determine whether the electronic evaporation tank is closed and enable the ventilation module to provide the respiratory support for a patient after determining that the electronic evaporation tank is closed, when the respiratory support mode, which is received by the human-machine interaction apparatus, is to provide respiratory support through the ventilation module.

29. The anesthesia machine according to claim 1 or claim 2, **characterized in that**, further comprising a three-way valve, wherein the three-way valve comprises a first port which is connected with the monitoring apparatus for flow amount, a second port which is connected with the anesthetic delivery apparatus, and a third port which is connected with the ventilation module; wherein the monitoring apparatus for flow amount comprises a fourth control valve and a fifth control valve;
the first port and the third port are connected with each other, and the fourth control valve and the fifth control valve are connected with the ventilation module, so as to enable the ventilation module to ventilate a patient at the first ventilation frequency, when the respiratory support mode, which is received by the anesthesia machine, is to provide respiratory support through the ventilation module;
the first port and the second port are connected with each other, and the fourth control valve and the fifth control valve are connected with the anesthetic delivery apparatus, so as to mix said first gas with the anesthetic at a second ventilation frequency for obtaining the second gas and enable the respiratory loop to ventilate a patient, when the respiratory support mode, which is received by the anesthesia machine, is to provide anesthesia respiratory support;
wherein the first ventilation frequency is higher than the second ventilation frequency.

30. The anesthesia machine according to claim 1, **characterized in that**, the first gas is at least an oxygen-containing gas, and the anesthesia machine further comprises an auxiliary gas supply module, which is configured to receive the first gas and use the first gas to provide respiratory support for a patient at a flow amount of 20-80 liters/minute.

31. The anesthesia machine according to any one of claims 1-30, **characterized in that**, the ventilation module is further configured to provide the respiratory support at the first ventilation frequency, a first ventilation control apparatus is configured to control a respiratory loop to provide the anesthesia respiratory support at a third ventilation frequency; wherein the first ventilation frequency is higher than the third ventilation frequency.

32. The anesthesia machine according to any one of claims 6, 8, 9, 11, 15, 16, 17, 20, 21 and 23, **characterized in that**, the second control valve is selected from a group consisting of: an electromagnetic valve, an electromagnetic servo valve, an electromagnetic switch valve, and an electromagnetic proportional valve.

33. The anesthesia machine according to claim 1, **characterized in that**, further comprising a gas source module which provides the first gas; wherein the gas source module is connected with the anesthetic delivery apparatus through the monitoring apparatus for flow amount; the ventilation module is connected with the gas source module.

34. The anesthesia machine according to claim 2, **characterized in that**, further comprising a first gas source module which provides the first gas, and a second gas source module which provides the third gas; wherein the second gas source module is connected with the anesthetic delivery apparatus through the monitoring apparatus for flow amount; the ventilation module is connected with the first gas source module.

35. The anesthesia machine according to any one of claims 1-3, **characterized in that**, further comprising a monitoring module for carbon dioxide, which is configured to detect a fractional concentration of end-tidal carbon dioxide for a patient, when the first ventilation control apparatus provides the anesthesia respiratory support for said patient, and/or detect a fractional concentration of end-tidal carbon dioxide for a patient, when the ventilation module provides the respiratory support for said patient.

36. An anesthesia machine, comprising a gas source interface, a respiratory loop, and a first ventilation control apparatus;
wherein one end of the gas source interface is connected with a gas source, the other end of the gas source interface is connected with an anesthetic delivery apparatus;
wherein the anesthetic delivery apparatus is configured to mix a gas, which is provided by the gas source, with an anesthetic, and deliver the mixed gas to the respiratory loop;
the first ventilation control apparatus is configured to control the respiratory loop to deliver the mixed gas to a patient, so as to provide anesthesia respiratory support for said patient;
**characterized in that**, the anesthesia machine further comprises:
a ventilation module, which is configured to provide respiratory support for a patient, by using the gas which is provided by the gas source; wherein the ventilation module comprises a gas delivery branch, which is configured to receive the gas which is provided by the gas source, and provide the respiratory support for said patient, at a ventilation frequency which is higher than 50 times/minute.

37. The anesthesia machine according to claim 36, **characterized in that**, the ventilation module further comprises a second control valve which is arranged inside the gas delivery branch, and an output port which is connected with the gas delivery branch; wherein the output port is further connected with a jet accessory, and the second control valve is controlled to switch an amount of flow at a switching frequency of at least 50 times/minute, so as to enable the gas, which is provided by the gas source and outputted from the jet accessory, which accessory is connected with the output port, to form a gas flow of high-frequency jet.

38. An anesthesia machine, comprising a gas source interface, a respiratory loop, and a first ventilation control apparatus;
wherein one end of the gas source interface is connected with a gas source, the other end of the gas source interface is connected with an anesthetic delivery apparatus;
wherein the anesthetic delivery apparatus is configured to mix a gas, which is provided by the gas source, with an anesthetic, and deliver the mixed gas to the respiratory loop;
the first ventilation control apparatus is configured to control the respiratory loop to deliver the mixed gas to a patient, so as to provide anesthesia respiratory support for said patient;
**characterized in that**, the anesthesia machine further comprises:
a ventilation module, which is connected with a jet accessory, and configured to receive and regulate a gas which is outputted by the gas source interface, so as to enable said gas to provide respiratory support for a patient in a jet gas flow after passing through the jet accessory.

39. An anesthesia machine, comprising a gas source interface, a respiratory loop, and a human-machine interaction apparatus;
wherein one end of the gas source interface is connected with a gas source, the other end of the gas source interface is connected with an anesthetic delivery apparatus; wherein the anesthetic delivery apparatus is configured to mix a third gas, which is provided by the gas source, with an anesthetic, and deliver to the respiratory loop a second gas which is obtained after the mixing;
the respiratory loop is configured to deliver the second gas to a patient, so as to provide anesthesia respiratory support for said patient;
the human-machine interaction apparatus is configured to receive, from a user, a respiratory support mode for a patient which is provided by the anesthesia machine;
**characterized in that**, the anesthesia machine further comprises a communication module, which is configured to communicate with an external ventilation apparatus; wherein the ventilation apparatus is configured to provide respiratory support for a patient, by using a first gas at a first ventilation frequency; wherein the first ventilation frequency is not zero;
wherein the anesthesia machine is further configured to enable the anesthesia machine to provide the anesthesia respiratory support for a patient, and/or configured to control, through the communication module, the ventilation apparatus to provide the respiratory support for a patient, according to the respiratory support mode which is received by the human-machine interaction apparatus.

40. The anesthesia machine according to claim 39, **characterized in that**, in order to enable the anesthesia machine to provide the anesthesia respiratory support for a patient, and/or control, through the communication module, the ventilation apparatus to provide the respiratory support for a patient, according to the respiratory support mode which is received by the human-machine interaction apparatus, the anesthesia machine is further configured to:
provide an area for displaying ventilation state and an area for switching ventilation mode on a same screen of a display interface of the human-machine interaction apparatus, wherein the area for switching ventilation mode is configured to display a first respiratory support mode and a second respiratory support mode for a user to select;
display, in the area for switching ventilation mode, a control window of the first respiratory support mode, so as to enable a user to set a ventilation parameter of the first respiratory support mode, when the first respiratory support mode is selected; and control, through the communication module, the ventilation apparatus based on said ventilation parameter, so as to enable the ventilation apparatus to provide the respiratory support for a patient according to said ventilation parameter;
display, in the area for switching ventilation mode, a control window of the second respiratory support mode, so as to enable a user to set a ventilation parameter of the second respiratory support mode, when the second respiratory support mode is selected; and to enable the anesthesia machine to provide the anesthesia respiratory support for a patient according to said ventilation parameter.

41. The anesthesia machine according to claim 40, **characterized in that**, in order to display, on the display interface of the human-machine interaction apparatus, the first respiratory support mode and the second respiratory support mode for a user to select, the anesthesia machine is further configured to:
simultaneously display, in the area for switching ventilation mode, the first respiratory support mode and the second respiratory support mode for a user to select; or
display the first respiratory support mode in the area for switching ventilation mode, and hide the second respiratory support mode.

42. The anesthesia machine according to claim 39, **characterized in that**, the anesthesia machine is further configured to display an operation interface of the anesthesia machine on a display interface of the human-machine interaction apparatus; wherein the operation interface of the anesthesia machine is for a user to select a second respiratory support mode and set a ventilation parameter of the second respiratory support mode;
the display interface of the human-machine interaction apparatus is further configured to display an operation interface of the ventilation apparatus; wherein the operation interface of the ventilation apparatus is for a user to select a first respiratory support mode and set a ventilation parameter of the first respiratory support mode;
when the first respiratory support mode is selected, the anesthesia machine is further configured to control, through the communication module, the ventilation apparatus, so as to enable the ventilation apparatus to provide the respiratory support for a patient;
when the second respiratory support mode is selected, the anesthesia machine is further configured to provide the anesthesia respiratory support for a patient.

43. The anesthesia machine according to claim 42, **characterized in that**, the anesthesia machine is further configured to display the operation interface of the ventilation apparatus on the display interface of the human-machine interaction apparatus, only after the anesthesia machine is in communicative connection with the ventilation apparatus through the communication module.

44. The anesthesia machine according to claim 39, **characterized in that**, the human-machine interaction apparatus of the anesthesia machine comprises a first human-machine interaction apparatus and a second human-machine interaction apparatus;
the anesthesia machine is further configured to display a control window of a first respiratory support mode on a display interface of the first human-machine interaction apparatus for a user to select the first respiratory support mode and set a ventilation parameter of the first respiratory support mode; display a control window of a second respiratory support mode on a display interface of the second human-machine interaction apparatus for a user to select the second respiratory support mode and set a ventilation parameter of the second respiratory support mode;
when the first respiratory support mode is selected, the anesthesia machine is further configured to control, through the communication module, the ventilation apparatus, so as to enable the ventilation apparatus to provide the respiratory support for a patient;
when the second respiratory support mode is selected, the anesthesia machine is further configured to provide the anesthesia respiratory support for a patient.

45. The anesthesia machine according to claim 44, **characterized in that**, the anesthesia machine is further configured to display the control window of the first respiratory support mode on the display interface of the first human-machine interaction apparatus, only after the anesthesia machine is in communicative connection with the ventilation apparatus through the communication module.

46. A ventilation control method for an anesthesia machine, **characterized in that**, comprising:
receiving, from a user, a respiratory support mode which is provided by the anesthesia machine;
when the received respiratory support mode is to provide anesthesia respiratory support: receiving a third gas, controlling the third gas to mix with an anesthetic to obtain a second gas, controlling the second gas to enter into a respiratory loop of the anesthesia machine, and controlling the respiratory loop to output the second gas, so as to provide the anesthesia respiratory support for a patient;
when the received respiratory support mode is to provide respiratory support through a ventilation module: receiving a first gas, and controlling the ventilation module to provide the respiratory support for a patient, by using the first gas at a flow rate within a single respiratory cycle, which flow rate is not fixed.
